# EUROPEAN PATENT SPECIFICATION

(11) **EP 1 374 910 B1**
(45) Date of publication and mention of the grant of the patent: **09.05.2007**
(21) Application number: 03013934.9
(22) Date of filing: 03.06.1996
(51) Int. Cl.: A61K 48/00, C12N 15/86, C12N 15/49, A61K 39/12, A61K 39/118

(54) **Recombinant raccoon pox viruses and their use as an effective vaccine against feline immunodeficiency virus infection**
Rekombinante Pockenviren des Waschbaers und deren Verwendung als effektiver Impfschutz gegen Infektion mit FIV
Poxvirus recombinés du raton laveur et leur utilisation en tant que vaccin efficace contre l'infection par le virus de l'immunodeficience féline

(30) Priority: 07.06.1995 US 482090
(43) Date of publication of application: 02.01.2004
(62) Divisional of application: 96917038.0
(73) Proprietor: Wyeth, Madison, New Jersey 0784 (US)
(72) Inventor: Wasmoen, Terri, Omaha, Nebraska 68164 (US); Chu, Hsien-Jue, Fort Dodge, Iowa 50501 (US); Chavez, Lloyd George, Highlands Ranch, Colorado 80126 (US)
(74) Representative: Denholm, Anna Marie

(56) References cited:
- EP-A- 0 652 287
- WO-A-92/15684
- WO-A-94/06471
- US-A- 5 266 313
- US-A- 5 413 927
- MORIKAWA S ET AL: "ANALYSES OF THE REQUIREMENTS FOR THE SYNTHESIS OF VIRUS-LIKE PARTICLES BY FELINE IMMUNODEFICIENCY VIRUS GAG USING BACULOVIRUS VECTORS" VIROLOGY, vol. 183, no. 1, 1991, pages 288-297, XP000600250 ISSN: 0042-6822
- WOJCIK JACEK: "Expression of feline immunodeficiency virus (FIV) gag gene in vaccinia virus vector" ACTA MICROBIOLOGICA POLONICA, vol. 44, no. 2, 1995, pages 191-196, XP009018052 ISSN: 0137-1320
- COLE G E ET AL: "RECOMBINANT FELINE HERPESVIRUSES EXPRESSING FELINE LEUKEMIA VIRUS ENVELOPE AND GAG PROTEINS" JOURNAL OF VIROLOGY, vol. 64, no. 10, 1990, pages 4930-4938, XP000601916 ISSN: 0022-538X
- FRANCHINI GENOVEFFA ET AL: "Highly attenuated HIV type 2 recombinant poxviruses, but not HIV-2 recombinant Salmonella vaccines, induce long-lasting protection in rhesus macaques" AIDS RESEARCH AND HUMAN RETROVIRUSES, vol. 11, no. 8, 1995, pages 909-920, XP002079473 ISSN: 0889-2229
- BAXBY, D.: "Recombinant poxvirus vaccines" REVIEWS IN MEDICAL MICROBIOLOGY, vol. 4, 1993, pages 80-88, XP000600230

## Description

### FIELD OF THE INVENTION

The present invention pertains to the prophylaxis of disease caused by feline immunodeficiency virus (FIV), using as vaccines recombinant raccoon poxviruses (RRPVs) expressing the *gag* and envelope proteins of FIV.

### BACKGROUND OF THE INVENTION

Feline immunodeficiency virus (FIV) infection is a significant health problem for domestic cats around the world. As in its human counterpart, infection with FIV causes a progressive disruption in immune function. In the acute phase of infection, the virus causes transient illness associated with symptoms such as lymphadenopathy, pyrexia, and neutropenia. Subsequently, an infected animal enters an asymptomatic phase of 1-2 years before clinical manifestations of immune deficiency become apparent, after which the mean survival time is usually less than one year.

FIV is a typical retrovirus that contains a single-stranded polyadenylated RNA genome, internal structural proteins derived from the *gag* gene product, and a lipid envelope containing membrane proteins derived from the *env* gene product (Bendinelli *et al.,* Clin.Microbiol. Rev. 8:87, 1995). The *gag* gene is translated into a primary product of about 50 kDa that is subsequently cleaved by a viral protease into the matrix, capsid, and nucleocapsid proteins. The *env* gene yields a primary translation product of 75-80 kDa (unglycosylated molecular weight); in infected cells, the precursor has an apparent molecular weight of. 145-150 kDa due to N-linked glycosylation. The *env* precursor is cleaved in the Golgi apparatus into the SU and TM proteins (also designated gp95 and gp40, respectively).

Most vaccines against FIV have failed to induce protective immunity. Ineffective vaccines have involved inactivated whole virus, fixed infected cells, recombinant CA and SU proteins, and a synthetic peptide corresponding to the V3 region of SU. In some cases, the vaccine actually enhanced infection after challenge. In one system, vaccination with paraformaldehyde-fixed virus or infected cells resulted in protective immunity (Yamamoto *et al.,* J. Virol. 67:601, 1993), but application of this approach by others was unsuccessful (Hosie *et al.,* in Abstracts of the International Symposium on Feline Retrovirus Research, 1993, page 50).

Wojcik Jacek (1995). 'Expression of Feline Immunodeficiency Virus (FIV) gag gene in vaccinia virus vector' *Acta Microbiologica Polonica 44(2)* describes the use of the FIV gag gene in a virus vaccinia virus vector. EP-A-0 652 1287 (American Home Products Corp.) 10 May 1995 discloses recombinant raccoon poxvirus useful as a vaccine against Feline Infectious Peritonitis Virus (FIPV).

Thus, there is a need in the art for an effective vaccine against FIV that utilizes the *gag* or *env* proteins, or fragments therefrom, as immunogens.

### SUMMARY OF THE INVENTION

The present invention pertains to the prevention or lessening of disease in cats caused by Feline Immunodeficiency Virus (FIV). Prevention or lessening of disease is understood to mean the amelioration of any symptoms, including immune system disruptions, that result from FIV infection.

The invention provides recombinant raccoon poxviruses having at least one internal gene comprising a DNA sequence that encodes FIV *gag* protein (*gag*)*,* FIV envelope protein (*env*)or a polypeptide consisting of amino acids 1-735 of FIV *env.*

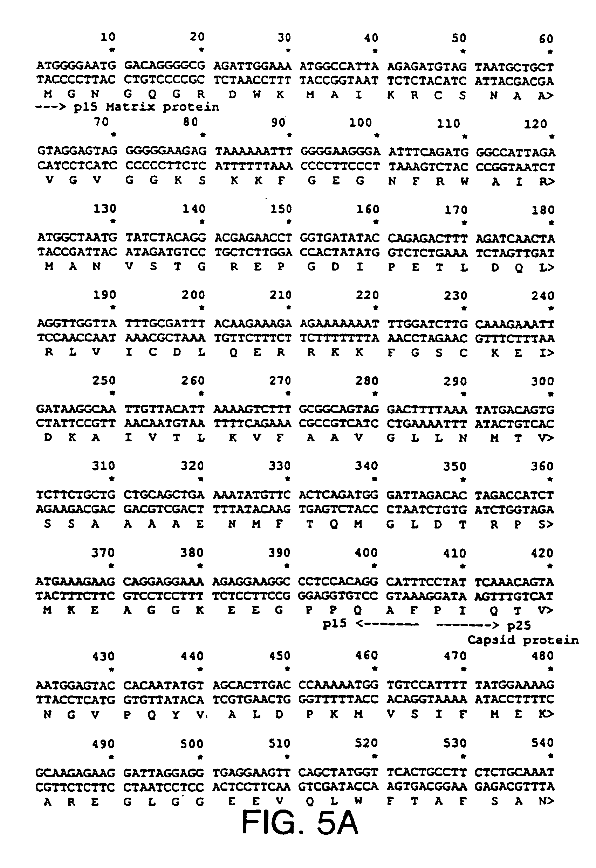

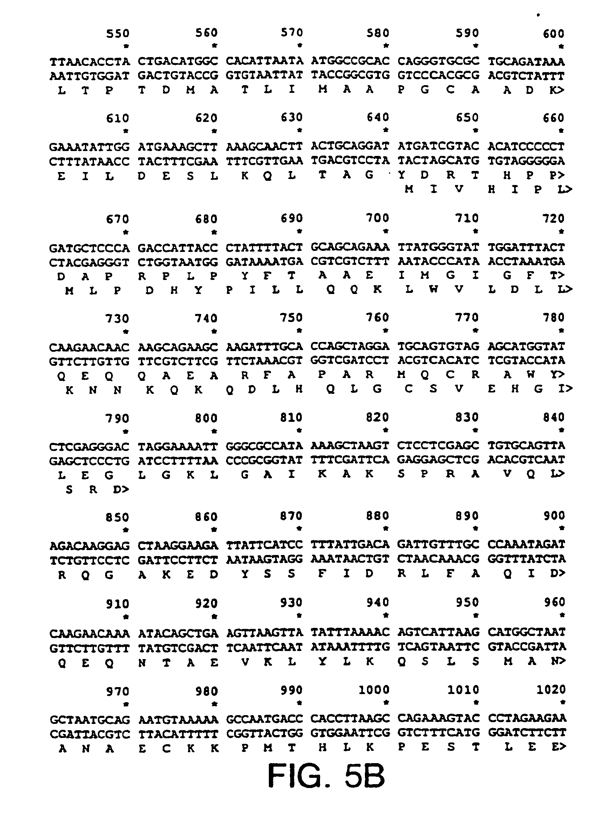

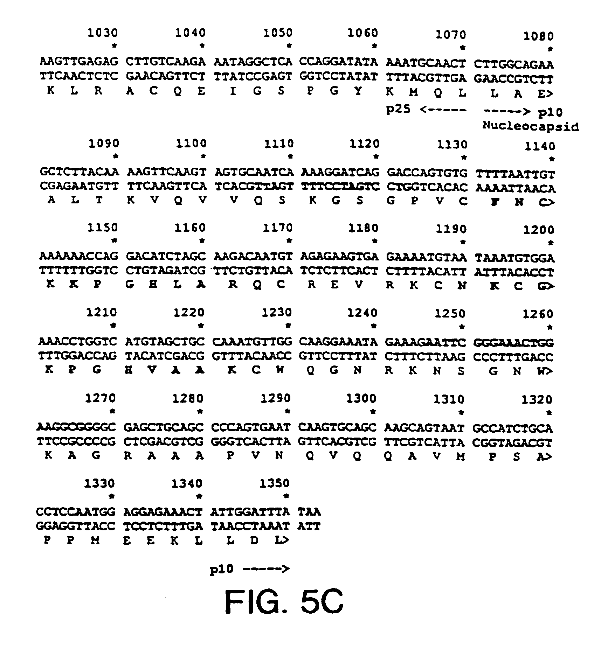

In one aspect the recombinant raccoon poxvirus has an internal gene encoding an FIV *gag* protein having the amino acid sequence as set out in Figure 5.
In another aspect the invention provides a vaccine comprising a recombinant raccoon poxvirus having at least one internal gene comprising a DNA sequence encoding *gag* protein of FIV and a pharmaceutically acceptable carrier or diluent. Preferably the internal gene encodes the FIV *gag* protein having the amino acid sequence set out in Figure 5. The vaccine may also comprise a pharmaceutically acceptable adjuvant.

The vaccine may further comprise immunogens derived from viruses selected from the group consisting of feline leukemia virus, feline panleucopenia virus, feline rhinotracheitis virus, feline calicivirus, feline infectious peritonitis virus, feline herpesvirus, feline enteric coronavirus, or mixtures thereof.

The vaccine may also comprise inactivated or attenuated feline *Chlamydia psittaci, Microsporum canis,* or mixtures thereof.

In a further aspect, the invention encompasses a use of a recombinant raccoon poxvirus having at least one internal gene comprising a DNA sequence encoding a *gag* protein of FIV, in the preparation of a vaccine for the prevention or lessening of disease caused by FIV.

In another aspect, the invention encompasses vaccines that comprise one or more of the FIV-expressing recombinant raccoon poxviruses described above, with a pharmaceutically acceptable carrier or diluent and a pharmaceutically acceptable adjuvant.

In yet another aspect, the invention provides methods for preventing or lessening disease caused by FIV, which is carried out by administering to a feline in need of such treatment the vaccines described above.

### BRIEF DESCRIPTION OF THE DRAWINGS

Figure 1 is a graphic illustration of the cloning strategy for the *env*elope gene of FIV.
Figure 2 is a diagrammatic representation of the structure of the recombinant FIV *env* gene in pSL-EnvABC.
Figure 3 shows the DNA [SEQ. ID No. 14] and protein [SEQ ID No. 12] sequence of the *env* gene of FIV.
Figure 4 is a graphic illustration of the pSL-WGag plasmid.
Figure 5 shows the DNA [SEQ ID No. 13] and protein [SEQ ID No. 11] sequence of the *gag* gene of FIV.
Figure 6 is a graphic illustration of the cloning strategy for construction of the raccoon poxvirus transfer plasmid pSC11-FIV *gag.*
Figure 7 is a graphic illustration of the cloning strategy for construction of the raccoon poxvirus transfer plasmid pSC11-FIV *Env.*
Figure 8 is a graphic illustration of the cloning strategy for construction of the raccoon poxvirus transfer plasmid pSC11-FIV *Env*AB.
Figure 9 is a table illustrating the detection of viremia and CD4:CD8 ratios in vaccinated and unvaccinated cats after FIV challenge.
Figure 10 is a table illustrating the preventable fraction for viremia and CD4:CD8 ratio changes in vaccinated and unvaccinated cats following FIV challenge.
Figure 11 is a table illustrating the clinical scores of vaccinated and unvaccinated cats after challenge with *Toxoplasma gondii.*

### DETAILED DESCRIPTION OF THE INVENTION

All patents, patent applications, and references cited herein are hereby incorporated by reference in their entirety. In the case of inconsistencies, the present disclosure, including definitions, will control.

The vaccine of the present invention may be prepared by creating recombinant raccoon poxviruses (RRPVs) containing a gene encoding the *gag* or *env* proteins of Feline Immunodeficiency Virus (FIV). *Gag* and *env* genes useful in practicing the present invention may be obtained by methods well-known in the art. In one embodiment, viral RNA is reverse- transcribed using endogenous or exogenous reverse transcriptase and the DNA is rendered double-stranded using DNA polymerase. The *gag* and *env*-encoding DNA segments are then recovered by restriction enzyme digestion and are amplified by cloning in *E. coli.* In another embodiment, FIV-infected cat cells serve as a source of FIV proviral DNA. In this embodiment, chromosomal DNA is isolated from the cells, and oligonucleotide primers are used to specifically amplify the *gag* and *env* genes or fragments therefrom using polymerase chain reaction techniques. This approach is broadly applicable to purifying *gag* and *env* genes from different FIV strains or isolates, since primers can be designed from non-polymorphic regions of the FIV genome.

FIV *gag* and *env* genes isolated by the above methods are first inserted into a transfer plasmid, and the recombinant plasmid is introduced into appropriate host cells that have been previously infected with a raccoon poxvirus. As a result, the DNA from the transfer plasmid is incorporated into the poxvirus DNA by homologous recombination, producing the RRPVs that are released from the cells.

DNA encoding the FIV *gag* or *env* proteins or fragments therefrom are inserted into a transfer plasmid downstream of a poxvirus promoter. In a preferred embodiment, the early/late 7.5 kD protein promoter of vaccinia, virus is used. However, alternate promoter elements could be used. The preferred transfer plasmid also contains a beta-galactosidase marker gene, which allows for selection and detection of the plasmid DNA sequences in recombinant viruses. It will be understood by those of ordinary skill in the art that alternate selectable marker genes, such as the neomycin resistance gene or the *E*. *coli gpt* gene or others, could be used to practice the invention. Flanking the inserted FIV gene and the selectable marker gene are thymidine kinase DNA sequences, which facilitate integration of the plasmid DNA sequences into the raccoon poxvirus DNA by homologous recombination.

Recombinant viruses expressing the FIV *gag* or *env* genes are prepared by first infecting a susceptible cell line (such as Vero [ATCC CCL 81], BSC- 1 [ATCC CCL 26], RAT-2 [ATCC CRL 1764], or CRFK [ATCC CCL 94]) with wild type raccoon poxvirus (ATCC VR-838 or similar isolates). Transfer plasmid DNA containing the FIV *gag* or *env* gene is then transfected into the infected cells using cationic liposome-mediated transfection, or other suitable techniques such as electroporation or calcium-phosphate precipitation. Raccoon poxviruses incorporate DNA from the transfer plasmid through homologous recombination with the thymidine kinase gene sequences present on the plasmid. Virus infection is allowed to proceed until cytopathic effects are noted in all cells.

Incorporation of the FIV *gag* or *env* gene into poxvirus DNA is accompanied by disruption of the viral thymidine kinase gene. Thus, recombinant virus may be selected for by the absence of a thymidine kinase gene; this is achieved by selective expansion on RAT-2 cells (tk-, ATCC CRL 1764) in the presence of 5-bromodeoxyuridine. Viruses containing a gene insert from the transfer plasmid are identified by blue plaque color when grown in the presence of a chromogenic substrate for beta-galactosidase such as X-gal.

Viral plaques that survive these selection and screening procedures are then subjected to several cycles of plaque purification. Subsequently, the presence of the *gag* or *env* genes is confirmed by polymerase chain reaction technology, and the presence of *gag* or *env* antigenic determinant is confirmed by immunoblot analysis using specific antibodies. These viruses are designated by RRPV-FIV *gag* and RRPV-FIV *env*, respectively.

In a further embodiment of the present invention, RRPVs can be produced that express less-than-full-length segments of the FIV *gag* and *env* proteins. The techniques used to engineer transfer plasmids encoding partial sequences of *env* and *gag* are well-known and widely used in the art, as are the methods for production and screening of RRPVs as detailed in this specification. For example, convenient restriction enzyme recognition sites can be used to obtain fragments of either gene, as described, e.g., Example 1 below. Alternatively, introduction of oligonucleotides containing a stop codon at various points along *gag* or *env* DNA will produce a nested set of carboxyterminal-truncated versions of that gene, which can then be incorporated into RRPVs. Furthermore, sequences that encode different domains on each protein may be recombined, using domains derived from different FIV strains or isolates. It will be apparent to one of ordinary skill in the art that systematic screening of such recombinant RRPVs can establish whether the intact protein, subfragments thereof or multi-strain recombinants thereof, are most preferred in practicing the present invention. Furthermore, as stated above, DNA encoding different fragments of *gag* and *env* can be used in a combination vaccine after incorporation into the same, or different, RRPVs.

For vaccine preparation, susceptible cells are grown in minimum essential media containing fetal bovine serum or a suitable media substitute. Cells are infected with recombinant raccoon poxvirus at a multiplicity of infection of 0.1 infectious units/cell or less. In this specification an infectious unit is defined as a Tissue Culture infectious Dose (TCID₅₀), an amount of virus yielding 50% infection under defined conditions. When cytopathology is noted in >90 % of the cells, the infected cells and extracellular fluids are harvested. The virus may be stored frozen (-50°C or colder) or lyophilized until the time of use. Compounds such as NZ-amine, dextrose, gelatin or others designed to stabilize the virus during freezing and lyophilization may be added. The virus may be concentrated using commercially available equipment.

Typically, the concentration of virus in the vaccine formulation will be a minimum of 10^{6.5} TCID₅₀ per dose, but will typically be in the range of 10^{7.0} to 10^{9.0} TCID₅₀ per dose. At the time of vaccination, the virus is thawed (if frozen) or reconstituted (if lyophilized) with a physiologically-acceptable carrier such as deionized water, saline, phosphate buffered saline, or the like. In one embodiment, a physiologically acceptable adjuvant such as, for example, EMA31, Adjuvant A, or combinations thereof, is added to the vaccine formulation. Non-limiting examples of suitable adjuvants include squalane and squalene (or other oils of animal origin); block copolymers such as Pluronic^{®} (L121) Saponin; detergents such as Tween^{®}-80; Quil^{®} A, mineral oils such as Drakeol^{®} or Marcol^{®}, vegetable oils such as peanut oil; Corynebacterium-derived adjuvants such as corynebacterium paryum; Propionibacterium-derived adjuvants such as Propionibacterium acne; Mycobacterium bovis (Bacillus Calinette and Guerinn, or BCG); interleukins such as interleukin 2 and interleukin-12; monokines such as interleukin 1; tumor necrosis factor; interferons such as gamma interferon; combinations such as saponin- aluminum hydroxide 1 or Quil^{®}-A aluminum hydroxide; liposomes; iscom adjuvant; mycobacterial cell wall extract; synthetic glycopeptides such as murainyl dipeptides or other derivatives; Avridine; Lipid A; dextran sulfate; DEAE-Dextran or DEAE-Dextran with aluminum phosphate; carboxypolymethylene, such as Carbopo^{®}; EMA; acrylic copolymer emulsions such as Neocryl^{®} A640 (e.g. U.S. Patent 5,047,238); vaccimia or ammal poxvirus proteins; subviral particle adjuvants such as orbivirus; cholera toxin; dimethyldiocledecylammonium bromide; or mixtures thereof.

EMA 31 (Monsanto, St. Louis, MO) is a linear ethylene/maleic copolymer with approximately equal amounts of ethylene and maleic anhydride, having an estimated average molecular weight of about 75,000 to 100,000. Adjuvant A is an adjuvant comprising a block copolymer, such as a polyoxypropylene-polyoxyethylene (POP-POE) block copolymer, preferably Pluronic^{®} L121 (e.g. U.S. Patent 4,772,466), and an organic component, such as a metabolizable oil, e.g. an unsaturated turpin hydrocarbon, preferably squalane (2,6,10,15,19,23-hexamethyltetracosane) or squalene. The vaccine may also include a non-ionic detergent or surfactant, preferably a polyoxyethylene sorbitan monooleate such as a Tween^{®} detergent, most preferably Tween^{®}-80, i.e. polyoxyethylene (20) sorbitan monooleate.

In this adjuvant mixture, the block copolymer, organic oil, and surfactant may be present in amounts ranging from about 10 to about 40 ml/L, about 20 to about 80 ml/L, and about 1.5 to about 6.5 ml/L, respectively. In a preferred embodiment of the stock adjuvant, the organic component is squalane present in an amount of about 40 mUL, the surfactant is polyoxyethylenesorbitan monooleate (Tween^{®}-80) present in an amount of about 3.2 ml/L, and the POP-POE block copolymer is Pluronic^{®} L121 present in an amount of about 20 ml/L. Pluronic^{®} L121 is a liquid copolymer at 15-40°C, where the polyoxypropylene (POP) component has a molecular weight of 3250 to 4000 and the polyoxyethylene (POE) component comprises about 10-20%, preferably 10%, of the total molecule.

Individual raccoon poxviruses expressing the *gag* or *env* genes may be mixed together for vaccination. Furthermore, the virus may be mixed with additional inactivated or attenuated viruses, bacteria, or fungi, or with immunogens derived from viruses, bacteria, or fungi such as feline leukemia virus, feline panleukopenia virus, feline rhinotracheitis virus, feline calicivirus, feline infectious peritonitis virus, feline *Chlanydia psittaci, Microsporum canis,* or others. In addition, antigens from the above-cited organisms may be incorporated into combination vaccines. These antigens may be purified from natural sources or from recombinant expression systems, or may comprise individual subunits of the antigen or synthetic peptides derived therefrom.

In a further embodiment of the present invention, live or inactivated RRPV virus-cell lysates can be incorporated into liposomes, or encapsulated in peptide-, protein-, or polysaccharide-based microcapsules prior to administration, using means that are known in the art. The vaccine of the present invention is administered to cats in volumes ranging from 0.5 to 5 milliliters. The vaccine can be administered to cats by subcutaneous, intramuscular, oral, intradermal, or intranasal routes. The number of injections and their temporal spacing may be varied. One to three vaccinations administered at intervals of one to three weeks are usually effective.

The efficacy of the vaccines of the present invention is assessed by the following methods. At about one month after the final vaccination, vaccinates and controls are each challenged with 3-20 cat ID₅₀ units, preferably 5 cat ID₅₀ units of FIV, preferably the NCSU₁ isolate (ATCC VR-2333). Whole blood is obtained from the animals immediately before challenge, and at intervals after challenge, for measurement of a) viremia and b) relative amounts of CD4 and CD8 lymphocytes.

Viremia is measured by isolating mononuclear cells from the blood, and co-culturing the cells with mononuclear cells from uninfected animals. After 7 days of culture, the culture supernatants are tested for FIV by enzyme-linked immunoassay (See Example 5 below).

The ratio of CD4 to CD8 lymphocytes in the circulation of vaccinates and controls is taken as a measure of immune function. Typically, FIV infection causes an inversion of the normal CD4:CD8 ratio of about 1.5-4.0 to a pathological ratio of about 0.5-1.0. The numbers of CD4 and CD8 lymphocytes are measured by flow cytometry using specific antibodies (see Example 5 below).

Another measure of immune function is to challenge vaccinates and controls with *Toxoplasma gondii* at 6-12 months after the final RRPV-FIV vaccination. Normally, the severity of *T*. *gondii-* induced disease symptoms is considerably exacerbated in FIV-infected cats relative to uninfected cats. The severity of the *T. gondii* effect is determined by scoring ocular discharge, nasal discharge, dyspnea, and fever.

It will be understood that amelioration of any of the symptoms of FIV infection is a desirable clinical goal. This includes a lessening of the dosage of medication used to treat FIV-induced symptoms.

The following examples are intended to illustrate the present invention without limitation thereof.

### Example 1: Cloning Of FIV gag and env Genes

### A. Isolation of Viral DNA

FIV strain NCSU-1 (designated "FIV-NCSU-1") was isolated from a naturally infected, feline leukemia virus-negative cat and has been described previously (Tompkins *et al.,* J. Am. Vet. Med. Assoc. 199: 1311, 1991. The virus was passed in a normal specific pathogen-free (SPF) cat (obtained from Liberty Laboratories, Waverly, NY). FIV-infected peripheral blood mononuclear cells (PBMC) were obtained from whole blood by separation on discontinuous percoll gradients. Briefly, anti-coagulated whole blood was layered over a two step gradient containing 43% Percoll^{™} (Pharmacia, Piscataway, NJ) over 62.5% Percoll^{™} in 0. 5M NaCI. Gradients were centrifuged at 400 x g for 5 minutes, followed by 800 x g for 20 minutes at 22°C. PBMC were harvested from the gradient interface and washed in phosphate buffered saline containing 5% fetal bovine serum. In parallel, PBMCs were isolated from normal cats.

FIV was propagated by co-culture of PBMCs from an FIV-infected cat with PBMCs from normal cats. The cells were maintained in RPMI 1640 media containing 10% fetal bovine serum, 2.5 x 10⁵ beta-mercaptoethenol, 2 mM L-glutamine, 5 µg/mL concanavalin A, and 20% conditioned media from MLA cells (ATCC TIB 201) as a source of interleukin-2 (IL-2).

Cat genomic DNA containing FIV-NCSU-1 proviral sequences was isolated from the cultured PBMCs by lysis of the cells with 0. 6 % sodium dodecyl sulfate (SDS) in 10 mM Tris-HCI, pH 7.5, 10 mM EDTA, followed by precipitation of chromosomal DNA by incubation overnight with 1 mM NaC1. The DNA was recovered by centrifugation at 10,000 r.p.m. (Beckman J2, JA-20 rotor) for 40 minutes. The DNA pellet was resuspended in a solution containing 10 inM Tris-HCl pH 7.5, 10 mM EDTA, 0.1% SDS buffer and digested with ribonuclease A (20 µg/ml) and proteinase K (0.2 mg/ml) at 50°C for 4 hours. DNA was then purified by sequential extraction with phenol, phenol: chloroform (1:1) and chloroform, and was recovered in pure form followed by ethanol precipitation.

### B. Cloning of FTV Envelope Gen

FIV-NCSU-1 envelope DNA sequences were cloned using polymerase chain reaction (PCR) methods as follows:

### Envelope Fragment A

The following oligonucleotides were used to amplify the 5' proximal segment of the *env* gene.
5'-TCGGATCCAACAATAATTATGGCAGAAGG-3' [SEQ ID No. 1] (Coding strand, 6252-V)
5'-AATCAGGTACAAAGTCACCGTTC-3' [SEQ ID No. 2] (Complementary strand, 6745-C)

Primer 6252-V corresponds to nucleotides 6252-6273 of FIV strain PPR (GenBank No. M36968) and primer 6745-C (underlined region) corresponds to nucleotides 6723-6745 of FIV strain 14 (GenBank No. 25381). The start codon for envelope protein translation is included in primer 6252-V. Primer 6252-V also has a synthetic BamHI restriction enzyme site near the 5' end to facilitate cloning. An AvrII site located at position 6719 also facilitates cloning. Envelope fragment A is 494 bp in length.

### Envelope Fragment B

The following oligonucleotides were used to amplify the middle segment of the *env* gene.
5'-TATAGAAGCACCCCAAGAAGAG-3' [SEQ ID No. 3] (Coding strand, 6637-V)
5'-CATTCCCCCAAAGTTATATTTC-3' [SEQ ID No. 4] (Complementary strand, 8469-C) Primers 6637-V and 8469-C correspond to nucleotides 6637-6659 and 8448-8469 of FIV 14 strain, respectively. An AvrII site at position 6719 and a Spe1 site at position 8288 facilitated cloning. Envelope fragment B is 1833 bp in length.

### Envelope Fragment C

The following oligonucleotides were used to amplify the 3' distal fragment of the *env* gene.
5'- TTAGTTACATTAGAGCATCAAG-3' [SEQ ID No. 5] (Coding strand, 8264-V)
5'-TTCTAGATCTTCAGGGTCCCAATACTC-3' [SEQ ID No. 6]
(Complementary strand, 9145-C)

Primer 8264-V corresponds to nucleotides 8264-8285 of FIV strain 14, and primer 9145-C (underlined region) corresponds to nucleotides 9126-9145 of FIV strain PPR. Primer 9145-C has a synthetic Bg1II site near the 5' end to facilitate cloning. An SpeI site located at position 8288 also facilitated cloning. Envelope fragment C is 880 bp in length.

In each case, PCR was performed for 35 cycles of 1 min 30 sec at 94°C, 2 min at 56°C, and 2 min at 72°C, followed by one cycle of 8 min at 72°C. Each envelope fragment was isolated by gel electrophoresis and cloned into plasmid PSL1190 using standard methods (Maniatis *et al.,* Molecular Cloning: A Laboratory Manual, 1982, Cold Spring Harbor Press).

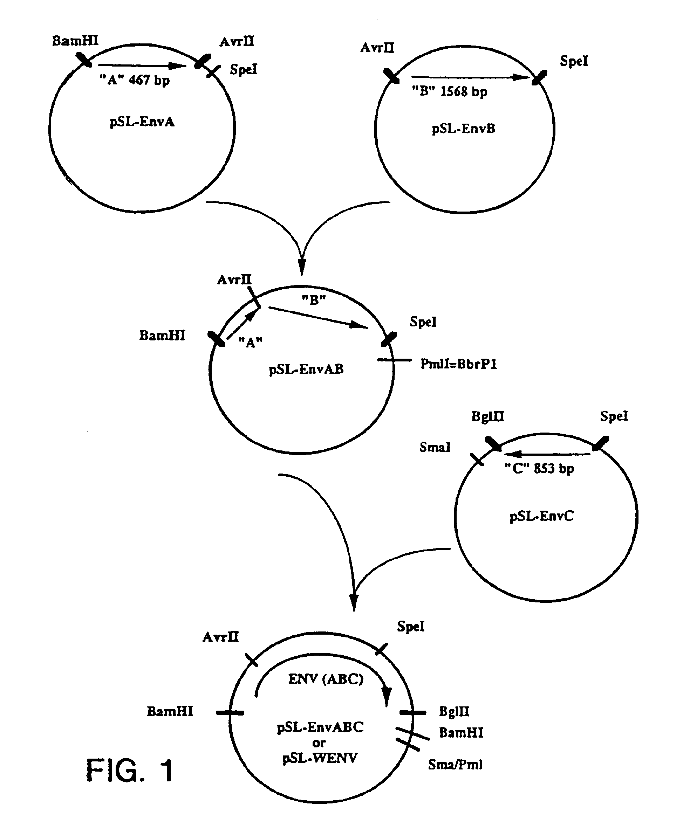

Initially, each fragment was cloned into pSL1190, after which the three fragments were spliced together to re-create a full length envelope gene. For this purpose, the Envelope A plasmid was digested with BamHI and AvrII, the envelope B plasmid was digested with AvrII and SpeI, and the envelope C plasmid was digested with SpeI and BgIII. Subsequently, the 1.5 kbp AvrII/SpeI envelope B fragment was ligated into pSL-EnvA that had been digested with AvrII and SpeI to create pSL-EnvAB (Figure 1). The *env*AB fragment codes for the entire surface membrane protein (SU) and the first 63 acids from the amino-terminus of the transmembrane protein (TM) of FIV-NCSU-1, i.e., amino acids 1-735 of *env.* However, *env*AB does not contain the transmembrane domain (TM).

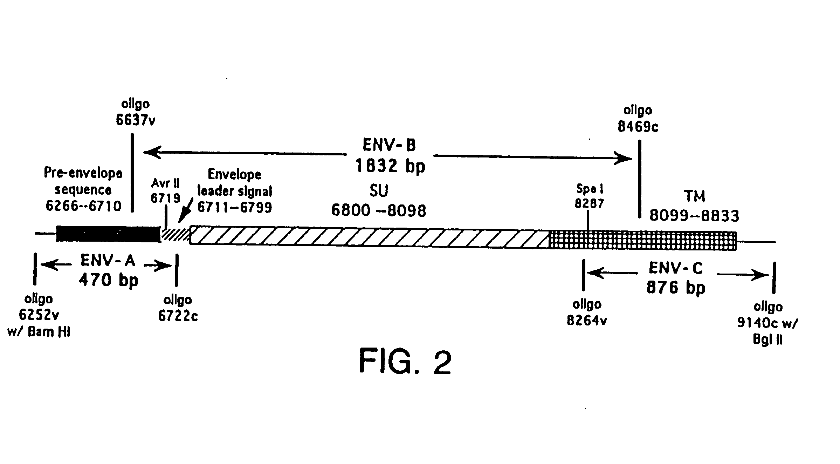

Next, the 0.9 kbp SpeI/Smal envelope C fragment from pSL-EnvC was ligated into pSL-EnvAB that had been digested with SpeI and BbrPI, to create pSL-EnvABC or pSL-WEnv (Figure 1). The WEnv fragment codes for the entire *env* open reading frame (SU and TM proteins) of FIV NCSU-1 (Figure 2).

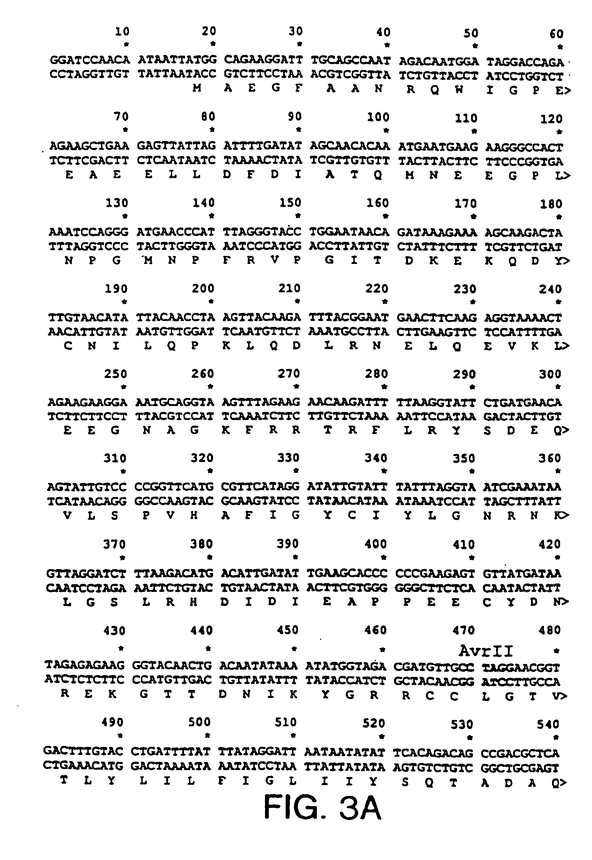

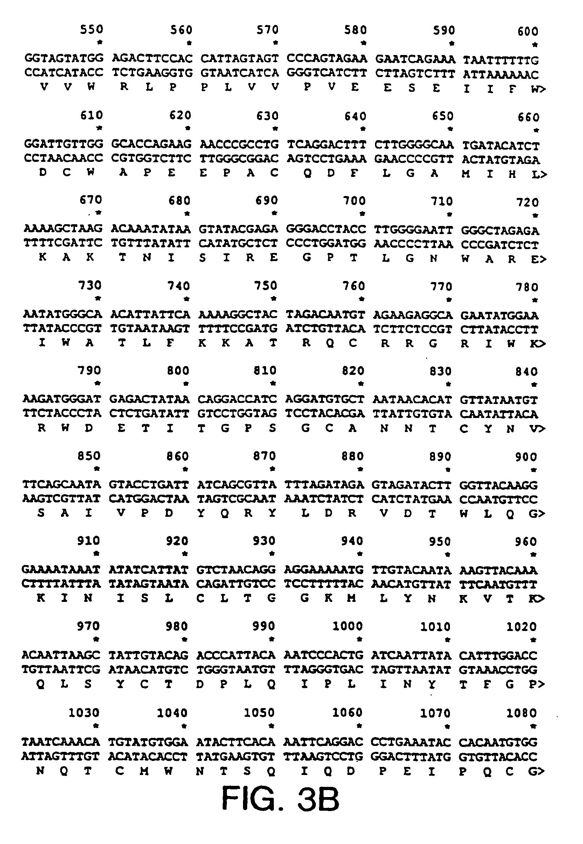

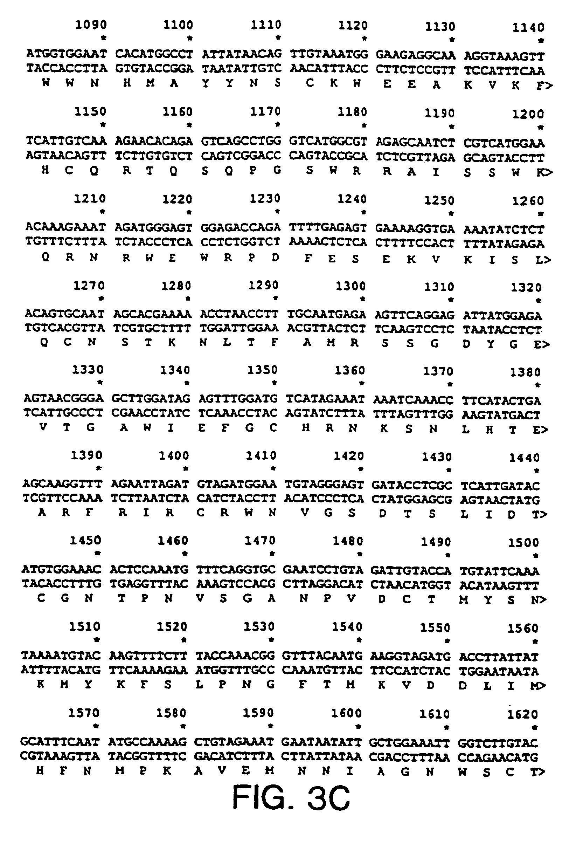

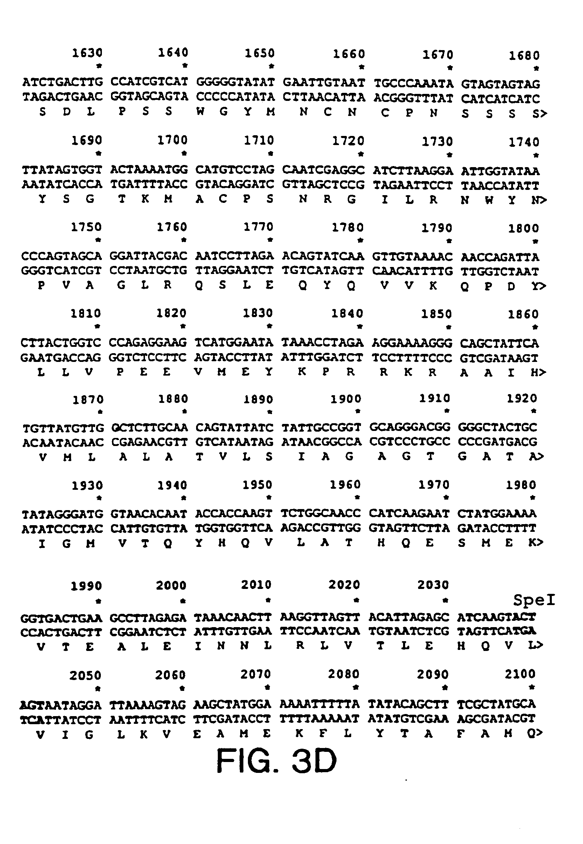

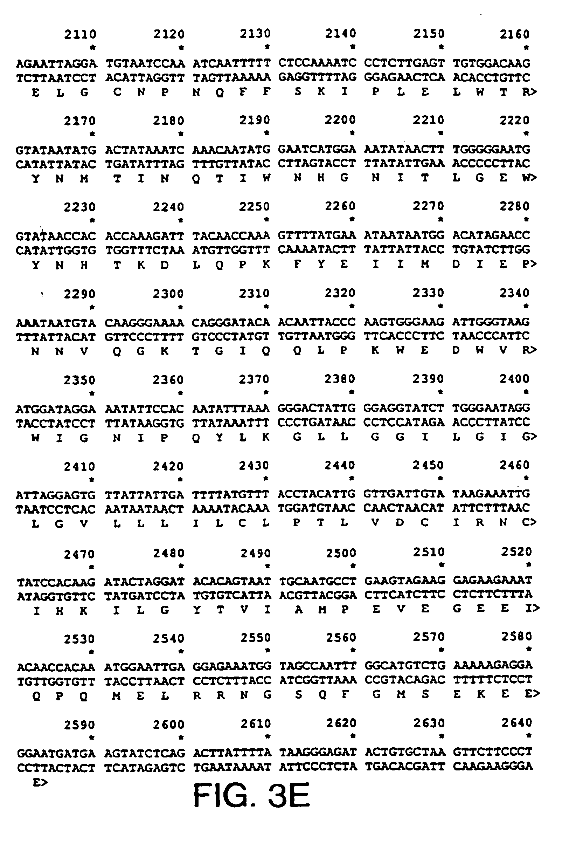

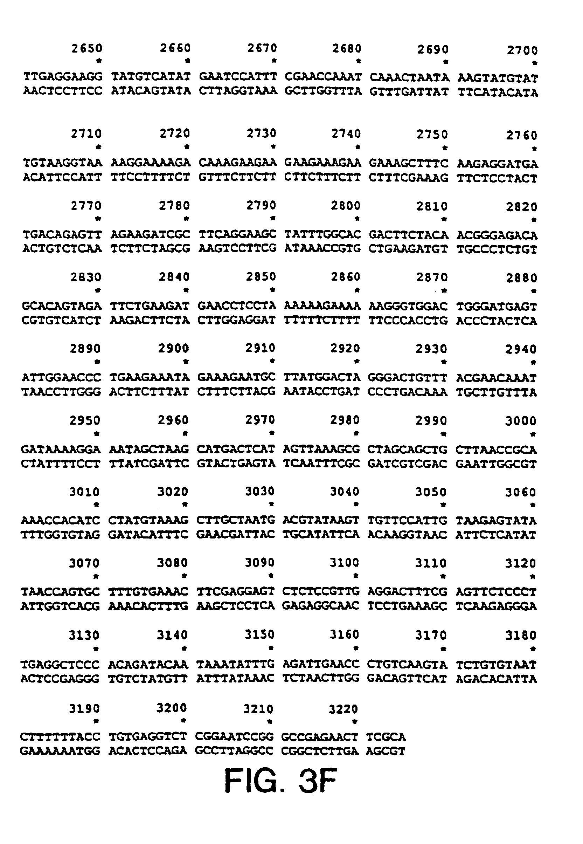

The subcloned genetic elements of FIV-NCSU-1 were sequenced using Sequenase Version 2.0 (United States Biochemical, Cleveland, OH)) as described for double-stranded DNA, and the reactions were analyzed using the ABI automated sequencer (Applied Biosystems, Foster City, CA). Both DNA strands were sequenced to confirm the results. The DNA sequences were analyzed using the MacVector DNA Analysis software (International Biotechnologies, Inc., New Haven, CT). The *env* DNA sequences were analyzed for open reading frames and compared to the previously published DNA sequences of other FIV isolates. The DNA and predicted amino acid sequences of *env* and *env*AB open reading frames of FIV-NCSU-1 are shown in Figure 3.

### C. Cloning of The FIV GAG Gene

The *gag* gene of FIV-NCSU, was amplified using PCR and the following oligonucleotide primers:
5'-CAATTCTAGAGAGACTCTACAGCAACATG-3' [SEQ ID No. 7] (Coding strand, 610-V)
5'-TAATAGATCTGGCCTCTTTTCTAATGATG-3' [SEQ ID No. 8]
   (Complementary strand, 2026-C)

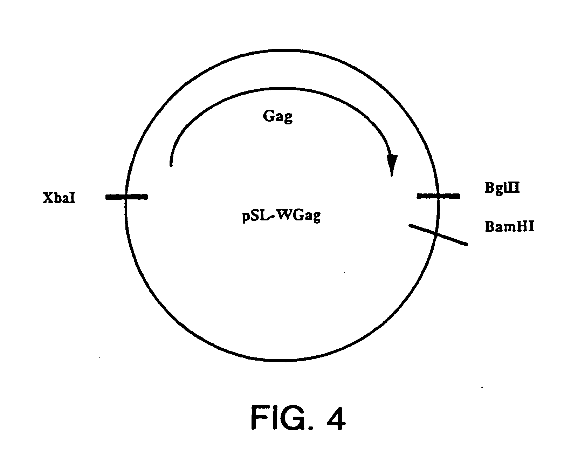

Primers 610-V and 2026-C correspond to nucleotides 610-630 and 2005-2026 of FIV 14 strain, respectively. Primers 610-V and 2026-C have XbaI and Bg1II restriction enzyme sites, respectively, near their 5' ends to facilitate cloning. The last three nucleotides of primer 610-V correspond to the start codon for *gag* protein translation. PCR was performed for 35 cycles of 1 min 30 sec at 94°C, 2 min at 56°C, and 2 min at 72°C, followed by one cycle of 4 min at 72°C. The 1.4 kbp DNA fragment containing the *gag* gene was purified by gel electrophoresis and cloned into the Xbal/Bg1II site of pSL1190 to form pSL-WGag (Figure 4). The DNA sequence of FIV-NCSU-1 *gag* is shown in Figure 5.

### Example 2. Preparation of Recombinant Raccoon Poxviruses

### A. Construction of Raccoon Poxviruses Transfer Plasmids

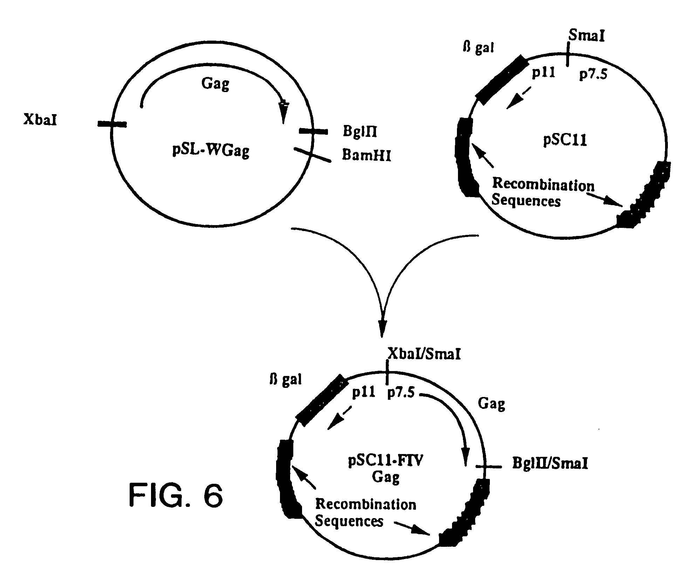

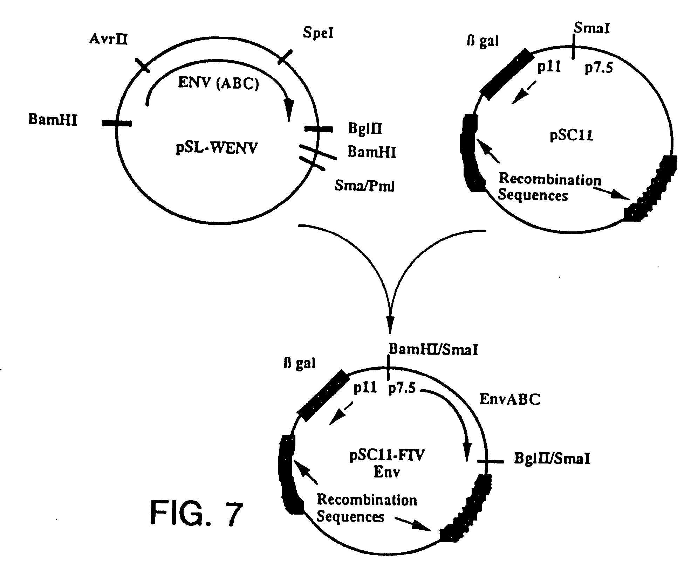

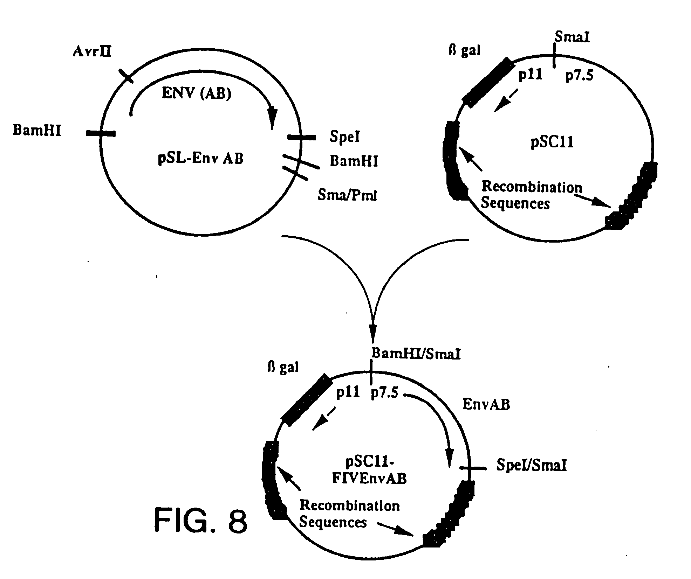

The DNA sequences encoding the *gag, env,* and *env*AB isolated as described in Example 1 were individually subcloned into the poxvirus transfer vector pSC11. The sequence of pSC11 is disclosed in co-pending U.S. patent application Serial No. 08/125,516, which is incorporated by reference. For this purpose, the 1.4 kb XbaI/BgIII fragment of pSL-WGag (Figure 6), the 2.9 kb BamHI/SpiI fragment of pSL-WEnv (Figure 7) and the 2.1 kb BamHI/SpeI fragment of pSL-EnvAB (Figure 8) were individually isolated and rendered blunt-ended with the Klenow fragment of DNA polymerase, after which each was individually cloned into the Smal site of pSC11.

### B. Preparation of Recombinant Raccoon Poxviruses

Recombinant raccoon pox viruses (RRPV) bearing the FIV *gag* and *env* genes were prepared as generally described for recombinant vaccinia viruses (Mackett and Smith, J Gen Virol 67:2067-2082, 1986) with some modifications. Monolayers of Vero cells (ATCC CCL 81) that were 80% confluent (approximately 5 x 10⁶ cells in 100 mm tissue culture dishes) were infected with wild-type raccoon pox virus (ATCC VR-838) at a multiplicity of infection (MOI) of 0.1 TCID₅₀/cell in 2 ml of MEM (Eagle's Minimum Essential Medium (Gibco BRL #410-1500) containing 0.05% lactalbumin hydrolysate and µg/ml gentamicin sulfate, adjusted to pH 7.2 with sodium bicarbonate) for 30-60 minutes at 37°C. The cells were then transfected with either pSC11-FIV *gag,* pSC11-FIV *env,* or pSC11-FIV *env* AB transfer plasmids by cationic liposome-mediated transfection using Transfectam® (Promega Corporation, Madison, Wisconsin) according to manufacturer's instructions. The cells/DNA-liposomes mixture was incubated in 3 ml of MEM containing 5% fetal bovine serum (FBS) overnight at 37°C (5% CO₂), after which the medium was replaced with 8 ml fresh MEM/5% FBS. The transfected cells were incubated at 37°C (5% CO₂) until greater than 80% of the cells showed cytopathic effects (approximately 3-4 days). The cells and culture media (viral-cell lysates) were then removed from the plates and subject to two cycles of freeze-thawing before storage at -70°C.

### C. Isolation of Recombinant Raccoon Pox Virus Carrying the FIV gag

### Gene

PIRPV carrying the FIV-NCSU₁ *gag* gene (RRPV-FIV *gag)* are isolated and purified from the pSCII-FIV gag/Vero cell transfection by standard viral plaque assay methods. Monolayers of Vero cells (50-80% confluent) in 100 mm tissue culture dishes were infected with 2 ml of 10-fold serial dilutions (10⁻¹ to 10⁻³ in MEM) of the viral-cell lysates. After incubation, for 1 hour at 37°C, the media are removed and the infected cells were overlaid with 8-10 ml of 1.25 % Noble agar containing MEM/5% FBS. The infected cells were then incubated for 3-4 days at 37°C (5% CO₂), and overlaid again with 4 ml of 1.25% Nobel agar containing 0.5X PBS and 600 ug/ml 5-bromo-4-chloro-3-indolyl-β-D-galactopyranoside (X-gal, United States Biochemical, Cleveland, Ohio). The plates were incubated at 37°C (5% CO₂) for 4-16 hours, until blue viral plaques β-galactosidase positive) were observed. The recombinant viral plaques were picked with sterile blunt needles attached to a 1 cc syringe, suspended in 0.5 ml of 0.25 mg/ml trypsin, vortexed vigorously, and incubated at 37°C for 15-30 minutes. The disrupted viral plaques were then inoculated onto 5x10⁵ Vero cells in T-25 cm² flasks and incubated at 37°C (5% CO₂) until greater than 80% CPE was observed. The viral-cell lysates containing RRPV-FIV *gag* were subjected to two cycles of freeze-thawing and stored at -70°C. Five individual RRPV-FIV gagclones were selected and plaqued purified four times as described above.

### D. Isolation of Recombinant Raccoon Pox Virus Carrying FIV envAB Gene

RRPV carrying the FIV-NCSU, envAB gene (RRPV-FIV envAB) were isolated and purified from the pSC 11 -FIV envAB/Vero cell transfection using the methods as described for RRPV-FIV *gag* with some slight modifications. Thymidine kinase deficient (tk-) raccoon pox viruses from the initial viral-cell lysates were selected on tk- Rat-2 cells (ATCC CRL 1764). This was performed by inoculating 1 ml of the initial viral-cell lysate onto a monolayer of Rat-2 cells in a T-75 cm² flask (approximately 5x10⁶ cells) containing 5-bromodeoxyuridine (BrdU) at 30 µg/ml in MEM. The infected monolayer was incubated at 37°C (5 % CO₂) for 3-4 days until greater than 70 % CPE was observed. The tk- viral-cell lysates were subjected to two cycles of freeze-thawing two times and stored at -70°C. RRPV-FIV envAB were isolated and purified from the tk- viral-cell lysates by the standard viral plaque assay as described above for RRPV-FIV *gag* on Vero cells. Five individual RRPV-FIV *env* AB clones were selected and plaque purified five times.

### Example 3: Characteristics of Recombinant FIV-Expressing Raccoon Pox Viruses

### A. Confirmation of FIV gag and envAB Genes in RRPV by Polymerase Chain Reaction

The presence of the FIV *gag* and *env*AB genes in the RRPVs was confirmed using PCR. 90 µl of a viral-cell lysate was incubated with 10 µl of 10X PCR lysis buffer (1X; 10 mM Tris-M buffer, pH 8.5, containing 50 mM KCI, 2.5 mM MgCl₂, 0.5 % Tween 20, 0.3 mg/ml Proteinase K) for 16 hours at 50°C, then boiled for minutes. 10 µl of this lysate was used as a template in the PCR reaction. PCR was performed in 100 µl of 10 mM Tris-HCL buffer, pH 8.3, containing 50 mM KCI, 200 uM of each dNTP, 1.5mM MgCl₂, 30 pmoles of each primer, and 2.5 Units of AmpliTaq® DNA polymerase (Perkin-Elmer Cetus, Norwalk, Cl). The primers used in the PCR for FIV *gag* were:
5'-TATGGAAAAGGCAAGAGAAGGAC-3'[SEQ ID No. 9]
5'-TCGAGATACCATGCTCTACACTG-3', [SEQ ID No. 10]
   corresponding to nucleotides 471-493 and 763-785 of the FIV *gag* open reading frame, respectively. The primers used in the PCR for FIV *envAB* were:
5'-TATGGAAAAGATGGGATGAGACTA-3' [SEQ ID No. 15]
5'-GTCACTTACCTTCATAGTAAACC-3' [SEQ ID No. 16] corresponding to nucleotides 857-880 and 1513-1535 of the FIV *env* open reading frame, respectively. The PCR amplifications were performed in a DNA Thermal Cycler (Perlein-Elmer Cetus) by first heating the reaction mixes to 94°C for denaturation, and then 35 cycles of 1 minute at 95°C, 1 minute at 55°C, and 2 minutes at 72°C, and a final incubation of 8 minutes at 72°C. 10 µl of the PCR products were analyzed by electrophoresis in a horizontal-submarine 4% NuSieve® agarose (FMC BioProducts, Rockland, ME) gel in TAE buffer (40 mM Tris base, 20 mM sodium acetate, 1 mm EDTA, pH 7.2) by applying 5 V/cm for 1-2 hours, and staining with ethidium bromide. PCR amplifications with the FIV *gag* and *env* primers gave expected DNA fragments of 314 and 678 nucleotides, respectively. PCR amplifications using the pSC 11 FIV *gag* and envAB transfer plasmids served as positive controls. PCR amplifications using wild-type raccoon pox virus-Vero cell lysates served as a negative control.

### B. Confirmation of RRPV FIV gag and envAB Protein Expression by Western Blot Analysis

Confluent monolayers of Vero cells in a T-25 cm² flask (1-2 x 10⁶ cells) were infected with clones of either RRPV-FIV *gag* or RRPV-FIV *env*AB at an M.O.I. of 1 to 10 TCID₅₀ per cell. The infected cells were incubated at 37°C (5% CO₂) for 2-3 days until approximately 80% CPE was observed. 20 µl of the viral-cell lysate was added to 5 µl of5X Laemmli sample buffer (0.3 M Tris-HCl buffer, pH 6.8, containing 5% SDS, 50% glycerol, 0.4% bromophenol blue, and 3% 2-mercaptoethanol) and heated at 95°C for 5 minutes. The denatured protein samples were separated by SDS/polyacrylamide electrophoresis using a 4-15% gradient polyacrylamide gel (Maniatis et al., Molecular Cloning: A Laboratory Manual, 1982, Cold Spring Harbor Press). After electrophoresis, the proteins were transferred to nitrocellulose filters (Bio-Rad Laboratories, Hercules, CA) by electrotransfer using a Bio-Rad transfer apparatus per manufacturer's instructions. The transfer was performed in 25 mM Tris-HCI buffer, containing 0.2 M glycine and 20% methanol, for 45 minutes at 50V with constant current. The blot was then screened for FIV *gag* and *env*AB proteins by immunoblot analysis as previously described (Davis *et al.,* Basic Methods in Molecular Biology, 1986, Elsevier Science Publishing Company, New York, NY) with some slight modifications. After transfer, the nitrocellulose blot was rinsed in phosphate buffer saline, pH 7.4, containing 0.1% Tween-20 (PBS-TW), and nonspecific sites were blocked by incubating the blot in PBS containing 1% bovine serum albumin (PBS- BSA) at 4°C overnight, followed by a 15 minute wash in PBS-TW. The blot was then incubated for 30 minutes at room temperature with goat anti-FIV IgG diluted 1:100 in PBS-TW containing 1% BSA (PBS-TW-BSA), followed by four 5 minute washes in PBS-TW. Next, the blot was incubated for 30 minutes at room temperature with a biotin-labeled mouse-anti-goat IgG antibody (secondary antibody) (Kirkegaard & Perry Laboratories Inc., Gaithersburg, MD) diluted 1:2000 in PBS-TW-BSA, followed by four 5-minute washes in PBS-TW. Antigen-antibody complexes were detected by, incubating the blot for 30 minutes at room temperature with horseradish peroxidase-conjugated streptavidin (Kirkegaard & Perry Laboratories Inc.) diluted 1:1000 in PBS-TW, washing four times for 5 minutes each in PBS-TW, and visualizing with peroxidase chromogenic substrate (Kirkegaard & Perry Laboratories Inc.). Sucrose-gradient purified FIV and wild-type raccoon pox virus/Vero cell lysates were used as the positive and negative controls for the immunoblot analysis, respectively.

Goat anti-FIV antibodies were prepared as follows. FIV NCSU₁, was grown in peripheral blood lymphocytes and concentrated using a hollow fiber apparatus to a concentration of about 10⁶ TCID₅₀/ml. The concentrated virus stock was mixed with an oil adjuvant such as OW3 in a ratio of 1:1 (v:v), and the emulsion was used to inoculate goats six times, at intervals of 3-4 weeks. At monthly intervals, the goats were bled and the serum was tested for the presence of anti-FIV antibodies.

### C. Confirmation of RRPV FIV gag and envAB Protein Expression by Immunofluorescence Assay

Confluent monolayers of Vero cells in 96-well plates (1-2 x 10⁴ cells/well) are infected with clones of either RRPV-FIV *gag* or RRPV-FIV *envAB* at a multiplicity of infection of 0.1 to 1.0 plaque forming units per cell. Cell infected with wild-type RCNV serve as a negative control. The infected cells are incubated at 37°C (5% CO₂) for 1 day until approximately 20% CPE is observed. The cells are then washed three times with PBS and fixed with 80% acetone at 4°C for ten minutes. Next, the cells are rehydrated with PBS and incubated with a monoclonal antibody (IgG) against either FIV *gag* or *env* surface membrane proteins for 30 minutes at room temperature. FIV antigen/FIV antibody complexes are detected using a FITC-conjugated goat anti-mouse IgG (Kirkegaard & Perry Laboratories Inc., Gaithersburg, MD) and fluorescence microscopy.

### D. Raccoon Poxvirus Titration

Virus preparations were pre-treated by dilution into an equal volume of 0.5% trypsin and incubation at 37°C for 30 minutes in order to release virus from inclusions. Serial dilutions (10-fold) of virus were then prepared in MEM and were inoculated (100 pd/well) in replicates of five onto Vero cells (1 x 10⁴ cells in 100 µl per well) in a 96 well plate. Plates were incubated for 3-5 days at 37°C (5% CO₂) and observed for cytopathology typical of raccoon poxvirus. Viral infectivity titers were calculated as 50% endpoints based on cytopathology using the methods of Reed and Muench (Reed and Muench, The American Journal of Hygiene 21: 493, 1938).

### Example 4: Preparation of Vaccines Based on Recombinant Pox Viruses

### A. Preparation of Master Seeds of RRPV-FIV gag and envAB Viruses

A single clone of each recombinant virus that showed significant recombinant protein expression (by the method described in Example 3 above) was selected for large-scale expansion to serve as a master seed virus. All recombinant virus expansions and titrations were done on Vero cells in MEM containing 2.5 % FBS. Each plaque-purified virus clone was expanded by inoculating a confluent monolayer of Vero cells in a T-150 cm² flask (1×10⁷ cells) with 1 ml of viral-cell lysate (approximately 10⁷ infectious virus particles), and incubating at 37°C (5% CO₂) until 100% CPE was observed (2-3 days). This viral-cell lysate served as a pre-master seed virus stock and was used to obtain the master seed virus. The pre-master seed of each recombinant virus was titrated on Vero cells and a TCID₅₀ was determined. The master seed viruses for RRPV- FIV *gag* and RRPV-FIV envAB were grown on Vero cells using an MOI of 0.01 and 0.1, respectively. Three roller bottles of confluent Vero cells were infected for each of the master seed viruses using MEM media supplemented with 2.5% fetal bovine serum and incubated for approximately 3 days at 37°C. Infected culture supernatant fluids were harvested, and seed viruses were aliquoted into 1.5 ml ampules, which were sealed and stored in a liquid nitrogen freezer.

### B. Preparation of Vaccines

Vero cells (3 x d) were seeded into 850 cm² roller bottles in 200 ml of growth media (MEM containing 0.5% lactalbumin hydrolysate and 5% heat-inactivated fetal bovine serum) and incubated for 18 hours at 37°C. The next day, the media were removed from the cells and replaced with 50 ml of RRPV-FIV *gag* at a multiplicity of infection of 0.01 in infection media (MEM containing 0.5% LAH and 2.5% heat-inactivated fetal bovine serum). The virus used was at the fourth passage beyond the master seed preparation. Virus was allowed to adsorb to the cells for 30 minutes at 37°C, after which the volume of medium was adjusted to 150 nm per roller bottle. Roller bottles were incubated at 37°C until 100% cytopathology was evident (3 days). A virus/cell lysate was then prepared and stored frozen (-70°C). The virus titer of RRPV-FIV *gag* was determined to be 10^{7.4} TCID₅₀/ml.

Recombinant raccoon poxviruses expressing the FIV *env*AB gene fragment were prepared in the same manner, except that a multiplicity of infection of 0. 1 was used. The virus was at the fourth passage beyond the master seed preparation. The RRPV-FIV envAB preparation was titered and found to contain 10^{6.4} TCID₅₀/ml.

Wild type raccoon poxvirus was grown using the same methods as described above. This virus preparation was found to contain 10^{7.7} TCID₅₀/ml.

### Example 5: Test of Efficacy of FIV Vaccines Based on Recombinant Pox Viruses

### A. Vaccination

Thirty 6-7 month old cats (specific pathogen-free, Harlan Sprague Dawley, Madison, WI), fifteen males and fifteen females, were used. Cats were divided into six groups and vaccinated twice, 21 days apart, as indicated below:

**Table 1. Assignment of Groups for Vaccination**

| Group | # Cats | Vaccine | Volume | Virus Dose (TID₅₀) | Route* |
|---|---|---|---|---|---|
| 1 | 5 | RRPV-FIV *gag* | 1 mL | 10^{7.4} | SC |
| 2 | 5 | RRPV-FIV *gag* | 1 mL | 10^{7.4} | IM |
| 3 | 5 | RRPV-FIV envAB | 3 mL | 10^{6.9} | SC |
| 4 | 5 | RRPV-FIV envAB | 2 mL | 10^{6.7} | IM |
| 5 | 5 | RRPV-FIV *gag*(1ml)+ | 4 mL | 10^{7.4} (*gag*) | SC |
| | | RRPV-FIV envAB | 3 mL | 10^{6.9} | SC |
| | | | | (*env*AB) | |
| 6 | 5 | Wild Type raccoon DOX virus | 1 mL | 10^{7.7} | SC |

| | | | | | |
|---|---|---|---|---|---|
| * SC = Subcutaneous Vaccination IM = Intramuscular Vaccination | | | | | |

### B. Experimental Design

Twenty-five cats were vaccinated with the recombinant raccoon poxvirus vaccines as indicated in Table 1. Five cats were administered a similar titer of wild type raccoon poxvirus to serve a negative controls. Two vaccinations were administered 21 days apart. Subcutaneous vaccinations were administered in the nape of the neck, and intramuscular vaccinations were administered in a rear thigh. Four weeks following the second vaccination, all cats were challenged with the NCSU-1 strain of FIV and monitored for viremia and evidence of lymphocyte population changes as described below. Eleven months following FIV challenge, cats in Groups 1, 2, 3, 4, and 6 were challenged with *Toxoplasma gondii* and monitored for 48 days for clinical signs of disease.

### C. FIV Challenge

Four weeks following the second vaccination, all of the cats were challenged subcutaneously with 10 cat ID₅₀ units of the NCSU₁, isolate of FIV(1:1000 dilution of lot # 021891). Whole blood was obtained from the cats prior to challenge, and periodically after challenge, in order to assess virus infection parameters as follows:

### 1. Detection of Viremia

Culture isolation of FIV was performed as described previously (Wasmoen et al*.,* Vet. Immuno. Immunopath. 35:83 1992). Mononuclear cells were isolated from whole blood using Percoll^{™} (Pharmacia Biotech, Piscataway NJ) gradients. 5 x 10⁵ cells from FIV-challenged cats were cultured with 1 x 10⁶ mononuclear cells isolated from uninfected cats. Cultures were fed with RPMI media every 7 days and supernatants tested for the presence of FIV by an enzyme-linked immunosorbent assay (ELISA) that detects FIV p25 antigen (Petcheck ELISA, IDDEXX, Portland ME).

### 2. Lymphocyte Subsets

Leukocytes were isolated from whole blood using Histopaque^{TM} (Sigma Chemical Company, St. Louis MO) and lymphocyte subsets quantitated by staining the cells with antibodies specific to CD4 (monoclonal antibody CAT30A), CD8 (monoclonal antibody FLSM 3.357), pan T lymphocytes (monoclonal antibody FLSM 1.572) or B lymphocytes (anti-cat IgG) followed by FACS analysis. These monoclonal antibodies are described elsewhere (Tompkins *et al.* Vet. Immunol. Immunopathol. 26:305, 1990) and the flow *cytometry procedure is the same as previously described (R. V. English et* al. J. Infect. Dis. 170:543, 1994). CD4:CD8 ratios were calculated.

### D. Toxoplasma gondii Challenge

Tacheozoites of the ME49 strain of *T. gondii* that were frozen in 10% glycerol were inoculated intraperitoneally into Swiss mice (Charles Rivers Laboratories) and serially passed in mice according to published procedures (Davidson *et al.,* Am. J. Pathol. W:1486, 1993). Tacheozoites harvested from peritoneal fluids of mice were enumerated using a hemacytometer. Cats were tranquilized using ketamine hydrochloride and inoculated with 50,000 fresh tachyzoites into the right common carotid artery that had been surgically isolated. Cats were monitored for clinical signs of disease, including ocular discharge, nasal discharge, dyspnea, fever, depression, and weight loss for 3 days prior to and 48 days following *T. gondii* inoculation.

Clinical signs follow *T. gondii* challenge were scored as follows:

| Clinical Sign | | Score |
|---|---|---|
| Fever | 103.0 to | 1 point per day |
| | 103.9°F | |
| | 104.0 to 104.9°F | 2 points per day |
| | ≥ 105.0°F | 3 points per day |
| (*Temperatures were not scored until* ≥°F *above baseline.)* | | |
| Depression/Lethargy | | 1 point per day |
| Dehydration | | 2 points per day |
| Nasal Discharge | | 1 point per day |
| Ocular Discharge | | 1 point per day |
| Respiratory Distress | | |
| Tachypnea | | 2 points per day |
| Dyspnea | | 4 points per day |

### E. RESULTS

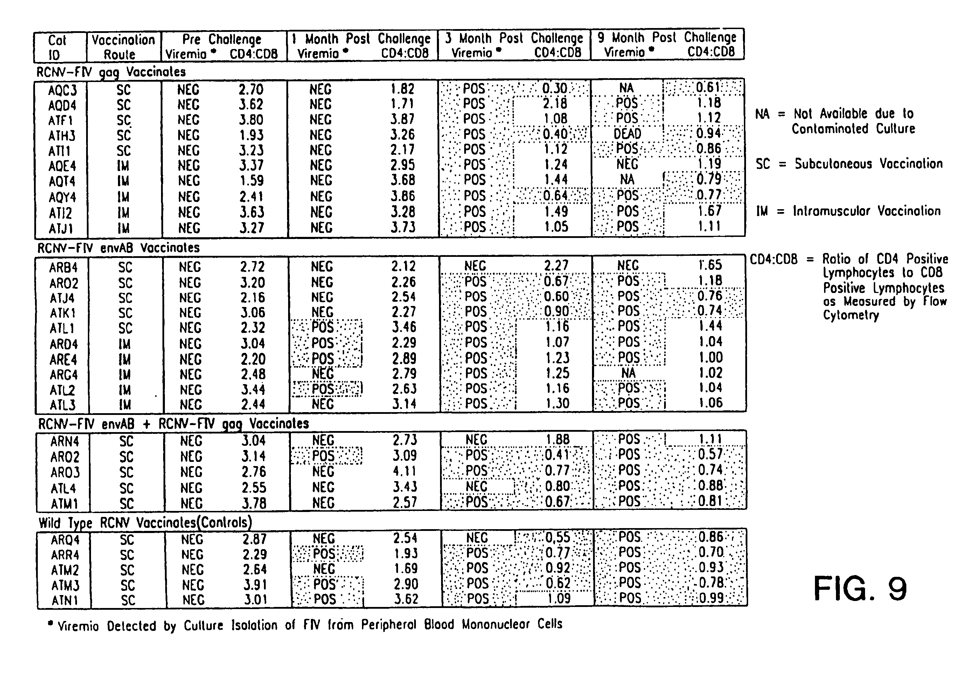

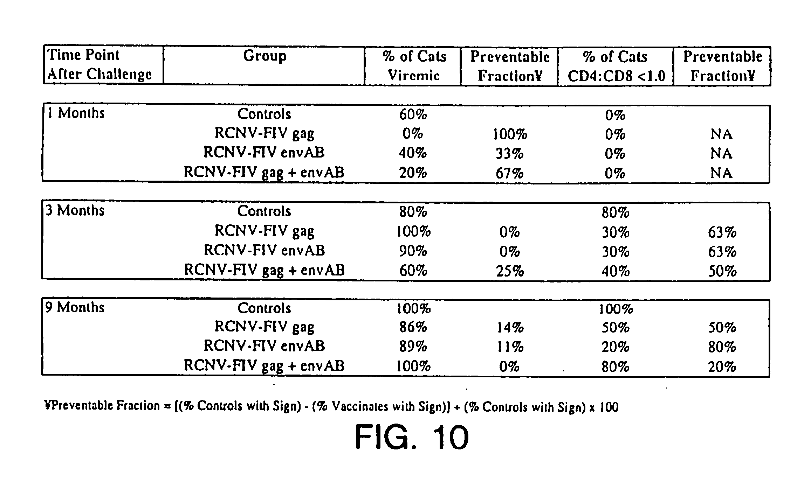

At one month following inoculation with the NCSU-1 strain of FIV, 60% of the control cats were found to be viremic (Figure 9). Cats vaccinated with RRPV-FIV *gag* were all negative for FIV, 40% of the cats vaccinated with RRPV-FIV *envAB* were virus positive, and 20% of the cats vaccinated with a combination of these two viruses were viremic (Figure 9). Therefore, the ability of the test vaccines to prevent viremia at this time point varied from 33 % to 100% (Figure 10).

At three months after FIV challenge, 80% of the control cats were found to be virus positive (Figure 9). Similarly, FIV could be isolated from peripheral blood mononuclear cells of nearly all vaccinated cats using this very sensitive method (Figure 9).

With respect to immune status, 80% of the control cats showed evidence of CD4:CD8 lymphocyte ratio inversions (i.e. ratios less than 1.0) at three months (Figure 9). In contrast, only 30% of the RRPV-gag vaccinated cats had evidence of significant CD4:CD8 inversions, and the RRPV-FIV envAB vaccinates were similarly protected from this lymphocyte subset change (Figure 9). Cats vaccinated with a combination of the two recombinant viruses were not significantly different from the controls (i.e. 80% showed CD4:CD8 inversions) at 3 months after challenge (Figure 9).

At 9 months after FIV challenge, 100% of the control cats were FIV infected, and all showed CD4:CD8 inversions (Figure 9). A large percentage of the vaccinated cats were also shown to be viremic at this time point. However, only 50% of the RRPV-FIV *gag* vaccinates and 20% of the RRPV-FIV *envAB* vaccinates showed evidence of CD4:CD8 inversions at this time point. *Therefore, these two vaccines showed a significant ability to prevent the CD4: CD8 lymphocyte ratio changes associated with FIV infection even though the cats appeared to be viremic (Figure 10).*

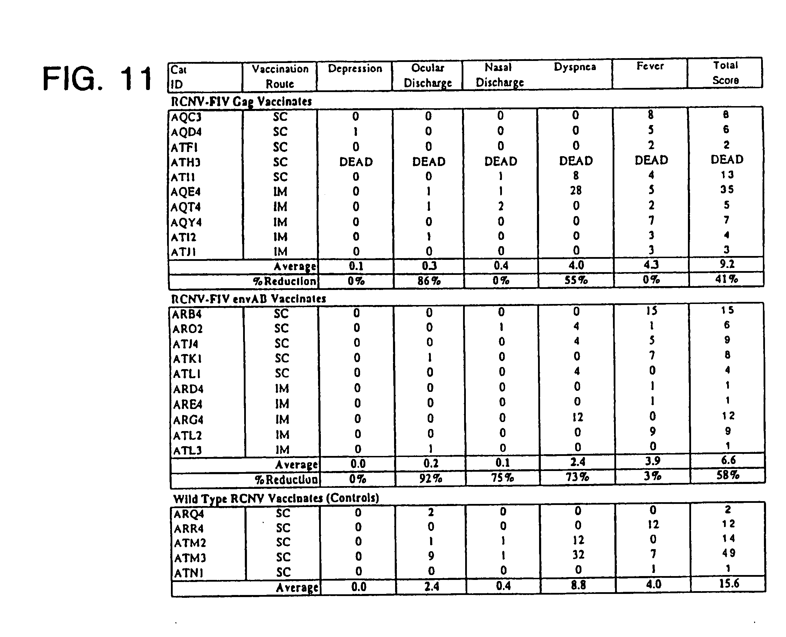

In order to determine whether CD4:CD8 lymphocyte subset inversions signified deterioration in the immune system of cats following FIV infection (and, conversely, that lack of inversion in vaccinates signified maintenance of immune function), vaccinated and control cats (from groups 1, 2, 3, 4, and 6) were challenged with *Toxoplasma gondii.* This parasite causes subclinical infections in normal cats, but has been reported to cause severe disease in cats that are immunocompromised due to FIV infection (Davidson. *et al.,* Am. J. Pathol. W: 1486, 1993). Following T. gondii challenge, control cats displayed ocular discharge, nasal discharge, dyspnea, and fever. The average total clinical score for control cats was 15.6 (Figure 11). By comparison, there was a 41 % reduction in clinical disease scores in RRPV-FIV *gag* vaccinated cats, related to reductions in clinical signs of ocular discharge and dyspnea (Figure 11). The clinical picture following *T. gondii* challenge was even less severe in RRPV-FIV envAB vaccinated cats. This group showed a 92% decrease in ocular signs, 75% decrease in nasal discharge, 73% reduction in dyspnea, and 58% decrease in overall clinical scores (Figure 11). Further, 80% of the control cats displayed weight loss in the first 14 days after challenge, compared to weight loss in only 44% of the RRPV-FIV *gag* vaccinates and 50% of the V-FIV *env*AB vaccinates. Therefore, control cats were more susceptible to induction of disease by this opportunistic pathogen than vaccinated cats.

These data suggest that vaccination altered the progression of clinical disease caused by this virus (i.e. induction of immune suppression). This is indicated by a lower rate of CD4:CD8 inversions in vaccinated cats and by a decreased susceptibility to infection with the opportunistic pathogen *T. gondii.*

## Claims

1. A recombinant raccoon poxvirus having at least one internal gene comprising a DNA sequence encoding the gag protein of Feline Immunodeficiency Virus (FIV) and having the amino acid sequence as set out in Figure 5.

2. A vaccine comprising a recombinant raccoon poxvirus according to claim 1, and a pharmaceutically acceptable carrier or diluent.

3. The vaccine of claim 2 further comprising a pharmaceutically acceptable adjuvant.

4. The vaccine of claim 2 further comprising immunogens derived from viruses selected from the group consisting of feline leukemia virus, feline panleucopenia virus, feline rhinotracheitis virus, feline calicivirus, feline infectious peritonitis virus, feline herpesvirus, feline enteric coronavirus, or mixtures thereof.

5. The vaccine of claim 2 further comprising inactivated or attenuated feline *Chlamydia psittaci, Microsporum canis,* or mixtures thereof.

6. Use of the recombinant raccoon poxvirus according to claim 1, in the preparation of a vaccine for the prevention of lessening of disease caused by Feline Immunodeficiency Virus (FIV)

7. Use according to claim 6, wherein said internal gene encodes the FIV gag protein having the amino acid sequence as set out in Figure 5.

## Patentansprüche

1. Rekombinantes Pockenvirus des Waschbärs mit mindestens einem internen Gen, das eine DNA-Sequenz umfasst, die das gag-Protein von felinem Immundefizienzvirus (FIV) kodiert und die wie in Figur 5 dargelegte Aminosäuresequenz aufweist.

2. Vakzin, das ein rekombinantes Pockenvirus des Waschbärs nach Anspruch 1 und einen pharmazeutisch unbedenklichen Trägerstoff oder ein pharmazeutisch unbedenkliches Verdünnungsmittel umfasst.

3. Vakzin nach Anspruch 2, das weiterhin ein pharmazeutisch unbedenkliches Adjuvans umfasst.

4. Vakzin nach Anspruch 2, das weiterhin Immunogene umfasst, die von Viren abgeleitet sind, die aus der Gruppe bestehend aus felinem Leukämievirus, felinem Panleukopenievirus, felinem Rhinotracheitisvirus, felinem Calicivirus, felinem infektiösem Peritonitisvirus, felinem Herpesvirus, felinem enterischem Coronavirus oder Mischungen davon ausgewählt sind.

5. Vakzin nach Anspruch 2, das weiterhin inaktiviertes oder attenuiertes felines *Chlamydia psittaci, Microsporum canis* oder Mischungen davon umfasst.

6. Verwendung des rekombinanten Pockenvirus des Waschbärs nach Anspruch 1 bei der Herstellung eines Vakzins zur Verhinderung oder Verminderung einer Erkrankung, die von felinem Immundefizienzvirus (FIV) verursacht wird.

7. Verwendung nach Anspruch 6, wobei das interne Gen das FIV-gag-Protein mit der wie in Figur 5 dargelegten Aminosäuresequenz kodiert.

## Revendications

1. Virus recombinant de variole du raton laveur ayant au moins un gène interne comprenant une séquence d'ADN codant pour la protéine gag du virus de l'immunodéficience féline (FIV) et ayant la séquence d'acides aminés telle qu'elle est représentée dans la figure 5.

2. Vaccin comprenant un virus recombinant de variole du raton laveur selon la revendication 1, et un support ou un diluant pharmaceutiquement acceptable.

3. Vaccin selon la revendication 2, comprenant en outre un adjuvant pharmaceutiquement acceptable.

4. Vaccin selon la revendication 2, comprenant en outre des immunogènes dérivés de virus choisis parmi le groupe constitué du virus de la leucémie féline, du virus de la panleucopénie féline, du virus de la rhinotrachéite féline, du calicivirus félin, du virus de la péritonite infectieuse féline, du virus de l'herpès félin, du coronavirus entérique félin, ou de leurs mélanges.

5. Vaccin selon la revendication 2, comprenant en outre une *Chlamydia psittaci* féline inactivée ou atténuée, un *Microsporum canis* félin inactivé ou atténué, ou leurs mélanges.

6. Utilisation du virus recombinant de variole du raton laveur selon la revendication 1, dans la préparation d'un vaccin destiné à la prévention ou l'atténuation d'une maladie causée par le virus de l'immunodéficience féline (FIV)

7. Utilisation selon la revendication 6, dans laquelle ledit gène interne code pour la protéine gag du FIV ayant la séquence d'acides aminés telle qu'elle est représentée dans la figure 5.


### SEQUENCE LISTING

(1) GENERAL INFORMATION:
   (i) APPLICANT: Wyeth
      Five Giralda Farms
      Madison, New Jersey 07940
      United States of America
   (ii) TITLE OF INVENTION: Recombinant Raccoon Pox Viruses and Their Use as an Effective Vaccine Against Feline Immunodeficiency Virus Infection
   (iii) NUMBER OF SEQUENCES: 16
   (iv) CORRESPONDENCE ADDRESS:
      (A) ADDRESSEE: Wyeth
      (B) STREET: Huntercombe Lane South
      (C) CITY: Taplow, Maidenhead
      (D) STATE: Berkshire
      (E) COUNTRY: UK
      (F) ZIP: SL6 OPH
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Ingham, Stephen H.
      (B) REGISTRATION NUMBER:
      (C) REFERENCE/DOCKET NUMBER: AHP-95059-01
   (ix) TELECOMMUNICATION INFORMATION:
      (A) TELEPHONE: 1628 413873
      (B) TELEFAX: 1628 799098
(2) INFORMATION FOR SEQ ID NO:1:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Feline immunodeficiency virus
      (B) STRAIN: PPR
   (viii) POSITION IN GENOME:
      (B) MAP POSITION: 6252-6273
      (C) UNITS: bp
   (xi) SEQUENCE DESCRIPTION: SEQ ID NO:1:
      TCGGATCCAA CAATAATTAT GGCAGAAGG 29
(2) INFORMATION FOR SEQ ID NO:2:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iv) ANTI-SENSE: YES
   (vi) ORIGINAL SOURCE:
      (A) ORGANISM: feline immunodeficiency virus
      (B) STRAIN: 14
   (viii) POSITION IN GENOME:
      (B) MAP POSITION: 6723-6745
      (C) UNITS: bp
   (xi) SEQUENCE DESCRIPTION: SEQ ID NO:2:
      AATCAGGTAC AAAGTCACCG TTC 23
(2) INFORMATION FOR SEQ ID NO:3:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (B) STRAIN: 14
   (viii) POSITION IN GENOME:
      (B) MAP POSITION: 6637-6659
      (C) UNITS: bp
   (xi) SEQUENCE DESCRIPTION: SEQ ID NO:3:
      TATAGAAGCA CCCCAAGAAG AG 22
(2) INFORMATION FOR SEQ ID NO:4:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iv) ANTI-SENSE: YES
   (vi) ORIGINAL SOURCE:
      (A) ORGANISM: feline immunodeficiency virus
      (B) STRAIN: 14
   (viii) POSITION IN GENOME:
      (B) MAP POSITION: 8448-8469
      (C) UNITS: bp
   (xi) SEQUENCE DESCRIPTION: SEQ ID NO:4:
      CATTCCCCCA AAGTTATATT TC 22
(2) INFORMATION FOR SEQ ID NO:5:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (B) STRAIN: 14
   (viii) POSITION IN GENOME:
      (B) MAP POSITION: 8264-8285
      (C) UNITS: bp
   (xi) SEQUENCE DESCRIPTION: SEQ ID NO:5:
      TTAGTTACAT TAGAGCATCA AG 22
(2) INFORMATION FOR SEQ ID NO:6:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (iv) ANTI-SENSE: YES
   (vi) ORIGINAL SOURCE:
      (A) ORGANISM: feline immunodeficiency virus
      (B) STRAIN: PPR
   (viii) POSITION IN GENOME:
      (B) MAP POSITION: 9126-9145
      (C) UNITS: bp
   (xi) SEQUENCE DESCRIPTION: SEQ ID NO:6:
      TTCTAGATCT TCAGGGTCCC AATACTC 27
(2) INFORMATION FOR SEQ ID NO:7:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (B) STRAIN: 14
   (viii) POSITION IN GENOME:
      (B) MAP POSITION: 610-630
      (C) UNITS: bp
   (xi) SEQUENCE DESCRIPTION: SEQ ID NO:7:
      CAATTCTAGA GAGACTCTAC AGCAACATG 29
(2) INFORMATION FOR SEQ ID NO:8:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (B) STRAIN: 14
   (viii) POSITION IN GENOME:
      (B) MAP POSITION: 2005-2026
      (C) UNITS: bp
   (xi) SEQUENCE DESCRIPTION: SEQ ID NO:8:
      TAATAGATCT GGCCTCTTTT CTAATGATG 29
(2) INFORMATION FOR SEQ ID NO:9:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (C) INDIVIDUAL ISOLATE: NCSU-1
   (viii) POSITION IN GENOME:
      (B) MAP POSITION: 471-493
      (C) UNITS: bp
   (xi) SEQUENCE DESCRIPTION: SEQ ID NO:9:
      TATGGAAAAG GCAAGAGAAG GAC 23
(2) INFORMATION FOR SEQ ID NO:10:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (C) INDIVIDUAL ISOLATE: NCSU-1
   (viii) POSITION IN GENOME:
      (B) MAP POSITION: 763-785
      (C) UNITS: bp
   (xi) SEQUENCE DESCRIPTION: SEQ ID NO:10:
      TCGAGATACC ATGCTCTACA CTG 23
(2) INFORMATION FOR SEQ ID NO:11:
   (i) SEQUENCE CHARACTERISTICS:
      (A) LENGTH: 450 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: feline immunodeficiency virus
      (C) INDIVIDUAL ISOLATE: NCSU-1
   (xi) SEQUENCE DESCRIPTION: SEQ ID NO:11:
   Met Gly Asn Gly Gln Gly Arg Asp Trp Lys Met Ala Ile Lys Arg Cys
   1               5                   10                  15
   Ser Asn Ala Ala Val Gly Val Gly Gly Lys Ser Lys Lys Phe Gly Glu
               20                  25                  30
   Gly Asn Phe Arg Trp Ala Ile Arg Met Ala Asn Val Ser Thr Gly Arg
           35                  40                  45
   Glu Pro Gly Asp Ile Pro Glu Thr Leu Asp Gln Leu Arg Leu Val Ile
       50                  55                  60
   Cys Asp Leu Gln Glu Arg Arg Lys Lys Phe Gly Ser Cys Lys Glu Ile
   65                  70                  75                  80
   Asp Lys Ala Ile Val Thr Leu Lys Val Phe Ala Ala Val Gly Leu Leu
   85 90 95
   Asn Met Thr Val Ser Ser Ala Ala Ala Ala Glu Asn Met Phe Thr Gln
   100 105 110
   Met Gly Leu Asp Thr Arg Pro Ser Met Lys Glu Ala Gly Gly Lys Glu
   115 120 125
   Glu Gly Pro Pro Gln Ala Phe Pro Ile Gln Thr Val Asn Gly Val Pro
   130 135 140
   Gln Tyr Val Ala Leu Asp Pro Lys Met Val Ser Ile Phe Met Glu Lys
   145 150 155 160
   Ala Arg Glu Gly Leu Gly Gly Glu Glu Val Gln Leu Trp Phe Thr Ala
   165 170 175
   Phe Ser Ala Asn Leu Thr Pro Thr Asp Met Ala Thr Leu Ile Met Ala
   180 185 190
   Ala Pro Gly Cys Ala Ala Asp Lys Glu Ile Leu Asp Glu Ser Leu Lys
   195 200 205
   Gln Leu Thr Ala Gly Tyr Asp Arg Thr His Pro Pro Asp Ala Pro Arg
   210 215 220
   Pro Leu Pro Tyr Phe Thr Ala Ala Glu Ile Met Gly Ile Gly Phe Thr
   225 230 235 240
   Gln Glu Gln Gln Ala Glu Ala Arg Phe Ala Pro Ala Arg Met Gln Cys
   245 250 255
   Arg Ala Trp Tyr Leu Glu Gly Leu Gly Lys Leu Gly Ala Ile Lys Ala
   260 265 270
   Lys Ser Pro Arg Ala Val Gln Leu Arg Gln Gly Ala Lys Glu Asp Tyr
   275 280 285
   Ser Ser Phe Ile Asp Arg Leu Phe Ala Gln Ile Asp Gln Glu Gln Asn
   290 295 300
   Thr Ala Glu Val Lys Leu Tyr Leu Lys Gln Ser Leu Ser Met Ala Asn
   305 310 315 320
   Ala Asn Ala Glu Cys Lys Lys Pro Met Thr His Leu Lys Pro Glu Ser
   325 330 335
   Thr Leu Glu Glu Lys Leu Arg Ala Cys Gln Glu Ile Gly Ser Pro Gly
   340 345 350
   Tyr Lys Met Gln Leu Leu Ala Glu Ala Leu Thr Lys Val Gln Val Val
   355 360 365
   Gln Ser Lys Gly Ser Gly Pro Val Cys Phe Asn Cys Lys Lys Pro Gly
       370                 375                 380
   His Leu Ala Arg Gln Cys Arg Glu Val Arg Lys Cys Asn Lys Cys Gly
   385                 390                 395                 400
   Lys Pro Gly His Val Ala Ala Lys Cys Trp Gln Gly Asn Arg Lys Asn
                   405                 410                 415
   Ser Gly Asn Trp Lys Ala Gly Arg Ala Ala Ala Pro Val Asn Gln Val
               420                 425                 430
   Gln Gln Ala Val Met Pro Ser Ala Pro Pro Met Glu Glu Lys Leu Leu
           435                 440                 445
   Asp Leu
       450
(2) INFORMATION FOR SEQ ID NO:12:
   (i) SEQUENCE CHARACTERISTICS:
      (A) LENGTH: 856 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: feline immunodeficiency virus
      (C) INDIVIDUAL ISOLATE: NCSU-1
   (xi) SEQUENCE DESCRIPTION: SEQ ID NO:12:
   Met Ala Glu Gly Phe Ala Ala Asn Arg Gln Trp Ile Gly Pro Glu Glu
   1               5                   10                  15
   Ala Glu Glu Leu Leu Asp Phe Asp Ile Ala Thr Gln Met Asn Glu Glu
               20                  25                  30
   Gly Pro Leu Asn Pro Gly Met Asn Pro Phe Arg Val Pro Gly Ile Thr
           35                  40                  45
   Asp Lys Glu Lys Gln Asp Tyr Cys Asn Ile Leu Gln Pro Lys Leu Gln
       50                  55                  60
   Asp Leu Arg Asn Glu Leu Gln Glu Val Lys Leu Glu Glu Gly Asn Ala
   65                  70                  75                  80
   Gly Lys Phe Arg Arg Thr Arg Phe Leu Arg Tyr Ser Asp Glu Gln Val
                   85                  90                  95
   Leu Ser Pro Val His Ala Phe Ile Gly Tyr Cys Ile Tyr Leu Gly Asn
               100                 105                 110
   Arg Asn Lys Leu Gly Ser Leu Arg His Asp Ile Asp Ile Glu Ala Pro
           115                 120                 125
   Pro Glu Glu Cys Tyr Asp Asn Arg Glu Lys Gly Thr Thr Asp Asn Ile
       130                 135                 140
   Lys Tyr Gly Arg Arg Cys Cys Leu Gly Thr Val Thr Leu Tyr Leu Ile
   145                 150                 155                 160
   Leu Phe Ile Gly Leu Ile Ile Tyr Ser Gln Thr Ala Asp Ala Gln Val
                   165                 170                 175
   Val Trp Arg Leu Pro Pro Leu Val Val Pro Val Glu Glu Ser Glu Ile
               180                 185                 190
   Ile Phe Trp Asp Cys Trp Ala Pro Glu Glu Pro Ala Cys Gln Asp Phe
           195                 200                 205
   Leu Gly Ala Met Ile His Leu Lys Ala Lys Thr Asn Ile Ser Ile Arg
       210                 215                 220
   Glu Gly Pro Thr Leu Gly Asn Trp Ala Arg Glu Ile Trp Ala Thr Leu
   225                 230                 235                 240
   Phe Lys Lys Ala Thr Arg Gln Cys Arg Arg Gly Arg Ile Trp Lys Arg
                   245                 250                 255
   Trp Asp Glu Thr Ile Thr Gly Pro Ser Gly Cys Ala Asn Asn Thr Cys
               260                 265                 270
   Tyr Asn Val Ser Ala Ile Val Pro Asp Tyr Gln Arg Tyr Leu Asp Arg
           275                 280                 285
   Val Asp Thr Trp Leu Gln Gly Lys Ile Asn Ile Ser Leu Cys Leu Thr
       290                 295                 300
   Gly Gly Lys Met Leu Tyr Asn Lys Val Thr Lys Gln Leu Ser Tyr Cys
   305                 310                 315                 320
   Thr Asp Pro Leu Gln Ile Pro Leu Ile Asn Tyr Thr Phe Gly Pro Asn
                   325                 330                 335
   Gln Thr Cys Met Trp Asn Thr Ser Gln Ile Gln Asp Pro Glu Ile Pro
               340                 345                 350
   Gln Cys Gly Trp Trp Asn His Met Ala Tyr Tyr Asn Ser Cys Lys Trp
           355                 360                 365
   Glu Glu Ala Lys Val Lys Phe His Cys Gln Arg Thr Gln Ser Gln Pro
       370                 375                 380
   Gly Ser Trp Arg Arg Ala Ile Ser Ser Trp Lys Gln Arg Asn Arg Trp
   385                 390                 395                 400
   Glu Trp Arg Pro Asp Phe Glu Ser Glu Lys Val Lys Ile Ser Leu Gln
                   405                 410                 415
   Cys Asn Ser Thr Lys Asn Leu Thr Phe Ala Met Arg Ser Ser Gly Asp
               420                 425                 430
   Tyr Gly Glu Val Thr Gly Ala Trp Ile Glu Phe Gly Cys His Arg Asn
           435                 440                 445
   Lys Ser Asn Leu His Thr Glu Ala Arg Phe Arg Ile Arg Cys Arg Trp
       450                 455                 460
   Asn Val Gly Ser Asp Thr Ser Leu Ile Asp Thr Cys Gly Asn Thr Pro
   465                 470                 475                 480
   Asn Val Ser Gly Ala Asn Pro Val Asp Cys Thr Met Tyr Ser Asn Lys
                   485                 490                 495
   Met Tyr Lys Phe Ser Leu Pro Asn Gly Phe Thr Met Lys Val Asp Asp
               500                 505                 510
   Leu Ile Met His Phe Asn Met Pro Lys Ala Val Glu Met Asn Asn Ile
           515                 520                 525
   Ala Gly Asn Trp Ser Cys Thr Ser Asp Leu Pro Ser Ser Trp Gly Tyr
       530                 535                 540
   Met Asn Cys Asn Cys Pro Asn Ser Ser Ser Ser Tyr Ser Gly Thr Lys
   545                 550                 555                 560
   Met Ala Cys Pro Ser Asn Arg Gly Ile Leu Arg Asn Trp Tyr Asn Pro
                   565                 570                 575
   Val Ala Gly Leu Arg Gln Ser Leu Glu Gln Tyr Gln Val Val Lys Gln
               580                 585                 590
   Asp Tyr Leu Leu Val Pro Glu Glu Val Met Glu Tyr Lys Pro Arg Arg
           595                 600                 605
   Pro Lys Arg Ala Ala Ile His Val Met Leu Ala Leu Ala Thr Val Leu
       610                 615                 620
   Ser Ile Ala Gly Ala Gly Thr Gly Ala Thr Ala Ile Gly Met Val Thr
   625                 630                 635                 640
   Gln Tyr His Gln Val Leu Ala Thr His Gln Glu Ser Met Glu Lys Val
                   645                 650                 655
   Thr Glu Ala Leu Glu Ile Asn Asn Leu Arg Leu Val Thr Leu Glu His
               660                 665                 670
   Gln Val Leu Val Ile Gly Leu Lys Val Glu Ala Met Glu Lys Phe Leu
           675                 680                 685
   Tyr Thr Ala Phe Ala Met Gln Glu Leu Gly Cys Asn Pro Asn Gln Phe
       690                 695                 700
   Phe Ser Lys Ile Pro Leu Glu Leu Trp Thr Arg Tyr Asn Met Thr Ile
   705                 710                 715                 720
   Asn Gln Thr Ile Trp Asn His Gly Asn Ile Thr Leu Gly Glu Trp Tyr
                   725                 730                 735
   Asn His Thr Lys Asp Leu Gln Pro Lys Phe Tyr Glu Ile Ile Met Asp
               740                 745                 750
   Ile Glu Pro Asn Asn Val Gln Gly Lys Thr Gly Ile Gln Gln Leu Pro
           755                 760                 765
   Lys Trp Glu Asp Trp Val Arg Trp Ile Gly Asn Ile Pro Gln Tyr Leu
       770                 775                 780
   Lys Gly Leu Leu Gly Gly Ile Leu Gly Ile Gly Leu Gly Val Leu Leu
   785                 790                 795                 800
   Leu Ile Leu Cys Leu Pro Thr Leu Val Asp Cys Ile Arg Asn Cys Ile
                   805                 810                 815
   His Lys Ile Leu Gly Tyr Thr Val Ile Ala Met Pro Glu Val Glu Gly
               820                 825                 830
   Glu Glu Ile Gln Pro Gln Met Glu Leu Arg Arg Asn Gly Ser Gln Phe
           835                 840                 845
   Gly Met Ser Glu Lys Glu Glu Glu
       850                 855
(2) INFORMATION FOR SEQ ID NO:13:
   (i) SEQUENCE CHARACTERISTICS:
      (A) LENGTH: 1353 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (C) INDIVIDUAL ISOLATE: NCSU-1
   (viii) POSITION IN GENOME:
      (B) MAP POSITION: 1-1353
      (C) UNITS: bp
   (xi) SEQUENCE DESCRIPTION: SEQ ID NO:13:
   ATGGGGAATG GACAGGGGCG AGATTGGAAA ATGGCCATTA AGAGATGTAG TAATGCTGCT    60
   GTAGGAGTAG GGGGGAAGAG TAAAAAATTT GGGGAAGGGA ATTTCAGATG GGCCATTAGA   120
   ATGGCTAATG TATCTACAGG ACGAGAACCT GGTGATATAC CAGAGACTTT AGATCAACTA   180
   AGGTTGGTTA TTTGCGATTT ACAAGAAAGA AGAAAAAAAT TTGGATCTTG CAAAGAAATT   240
   GATAAGGCAA TTGTTACATT AAAAGTCTTT GCGGCAGTAG GACTTTTAAA TATGACAGTG   300
   TCTTCTGCTG CTGCAGCTGA AAATATGTTC ACTCAGATGG GATTAGACAC TAGACCATCT   360
   ATGAAAGAAG CAGGAGGAAA AGAGGAAGGC CCTCCACAGG CATTTCCTAT TCAAACAGTA   420
   AATGGAGTAC CACAATATGT AGCACTTGAC CCAAAAATGG TGTCCATTTT TATGGAAAAG   480
   GCAAGAGAAG GATTAGGAGG TGAGGAAGTT CAGCTATGGT TCACTGCCTT CTCTGCAAAT   540
   TTAACACCTA CTGACATGGC CACATTAATA ATGGCCGCAC CAGGGTGCGC TGCAGATAAA   600
   GAAATATTGG ATGAAAGCTT AAAGCAACTT ACTGCAGGAT ATGATCGTAC ACATCCCCCT   660
   GATGCTCCCA GACCATTACC CTATTTTACT GCAGCAGAAA TTATGGGTAT TGGATTTACT   720
   CAAGAACAAC AAGCAGAAGC AAGATTTGCA CCAGCTAGGA TGCAGTGTAG AGCATGGTAT   780
   CTCGAGGGAC TAGGAAAATT GGGCGCCATA AAAGCTAAGT CTCCTCGAGC TGTGCAGTTA   840
   AGACAAGGAG CTAAGGAAGA TTATTCATCC TTTATTGACA GATTGTTTGC CCAAATAGAT   900
   CAAGAACAAA ATACAGCTGA AGTTAAGTTA TATTTAAAAC AGTCATTAAG CATGGCTAAT   960
   GCTAATGCAG AATGTAAAAA GCCAATGACC CACCTTAAGC CAGAAAGTAC CCTAGAAGAA  1020
   AAGTTGAGAG CTTGTCAAGA AATAGGCTCA CCAGGATATA AAATGCAACT CTTGGCAGAA  1080
   GCTCTTACAA AAGTTCAAGT AGTGCAATCA AAAGGATCAG GACCAGTGTG TTTTAATTGT  1140
   AAAAAACCAG GACATCTAGC AAGACAATGT AGAGAAGTGA GAAAATGTAA TAAATGTGGA  1200
   AAACCTGGTC ATGTAGCTGC CAAATGTTGG CAAGGAAATA GAAAGAATTC GGGAAACTGG  1260
   AAGGCGGGGC GAGCTGCAGC CCCAGTGAAT CAAGTGCAGC AAGCAGTAAT GCCATCTGCA  1320
   CCTCCAATGG AGGAGAAACT ATTGGATTTA TAA                               1353
(2) INFORMATION FOR SEQ ID NO:14:
   (i) SEQUENCE CHARACTERISTICS:
      (A) LENGTH: 3225 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (C) INDIVIDUAL ISOLATE: NCSU-1
   (viii) POSITION IN GENOME:
      (B) MAP POSITION: 1-3225
      (C) UNITS: bp
   (xi) SEQUENCE DESCRIPTION: SEQ ID NO:14:
   GGATCCAACA ATAATTATGG CAGAAGGATT TGCAGCCAAT AGACAATGGA TAGGACCAGA     60
   AGAAGCTGAA GAGTTATTAG ATTTTGATAT AGCAACACAA ATGAATGAAG AAGGGCCACT    120
   AAATCCAGGG ATGAACCCAT TTAGGGTACC TGGAATAACA GATAAAGAAA AGCAAGACTA    180
   TTGTAACATA TTACAACCTA AGTTACAAGA TTTACGGAAT GAACTTCAAG AGGTAAAACT    240
   AGAAGAAGGA AATGCAGGTA AGTTTAGAAG AACAAGATTT TTAAGGTATT CTGATGAACA    300
   AGTATTGTCC CCGGTTCATG CGTTCATAGG ATATTGTATT TATTTAGGTA ATCGAAATAA    360
   GTTAGGATCT TTAAGACATG ACATTGATAT TGAAGCACCC CCCGAAGAGT GTTATGATAA    420
   TAGAGAGAAG GGTACAACTG ACAATATAAA ATATGGTAGA CGATGTTGCC TAGGAACGGT    480
   GACTTTGTAC CTGATTTTAT TTATAGGATT AATAATATAT TCACAGACAG CCGACGCTCA    540
   GGTAGTATGG AGACTTCCAC CATTAGTAGT CCCAGTAGAA GAATCAGAAA TAATTTTTTG    600
   GGATTGTTGG GCACCAGAAG AACCCGCCTG TCAGGACTTT CTTGGGGCAA TGATACATCT    660
   AAAAGCTAAG ACAAATATAA GTATACGAGA GGGACCTACC TTGGGGAATT GGGCTAGAGA    720
   AATATGGGCA ACATTATTCA AAAAGGCTAC TAGACAATGT AGAAGAGGCA GAATATGGAA    780
   AAGATGGGAT GAGACTATAA CAGGACCATC AGGATGTGCT AATAACACAT GTTATAATGT    840
   TTCAGCAATA GTACCTGATT ATCAGCGTTA TTTAGATAGA GTAGATACTT GGTTACAAGG    900
   GAAAATAAAT ATATCATTAT GTCTAACAGG AGGAAAAATG TTGTACAATA AAGTTACAAA    960
   ACAATTAAGC TATTGTACAG ACCCATTACA AATCCCACTG ATCAATTATA CATTTGGACC   1020
   TAATCAAACA TGTATGTGGA ATACTTCACA AATTCAGGAC CCTGAAATAC CACAATGTGG   1080
   ATGGTGGAAT CACATGGCCT ATTATAACAG TTGTAAATGG GAAGAGGCAA AGGTAAAGTT   1140
   TCATTGTCAA AGAACACAGA GTCAGCCTGG GTCATGGCGT AGAGCAATCT CGTCATGGAA    1200
   ACAAAGAAAT AGATGGGAGT GGAGACCAGA TTTTGAGAGT GAAAAGGTGA AAATATCTCT    1260
   ACAGTGCAAT AGCACGAAAA ACCTAACCTT TGCAATGAGA AGTTCAGGAG ATTATGGAGA    1320
   AGTAACGGGA GCTTGGATAG AGTTTGGATG TCATAGAAAT AAATCAAACC TTCATACTGA    1380
   AGCAAGGTTT AGAATTAGAT GTAGATGGAA TGTAGGGAGT GATACCTCGC TCATTGATAC    1440
   ATGTGGAAAC ACTCCAAATG TTTCAGGTGC GAATCCTGTA GATTGTACCA TGTATTCAAA    1500
   TAAAATGTAC AAGTTTTCTT TACCAAACGG GTTTACAATG AAGGTAGATG ACCTTATTAT    1560
   GCATTTCAAT ATGCCAAAAG CTGTAGAAAT GAATAATATT GCTGGAAATT GGTCTTGTAC    1620
   ATCTGACTTG CCATCGTCAT GGGGGTATAT GAATTGTAAT TGCCCAAATA GTAGTAGTAG    1680
   TTATAGTGGT ACTAAAATGG CATGTCCTAG CAATCGAGGC ATCTTAAGGA ATTGGTATAA    1740
   CCCAGTAGCA GGATTACGAC AATCCTTAGA ACAGTATCAA GTTGTAAAAC AACCAGATTA    1800
   CTTACTGGTC CCAGAGGAAG TCATGGAATA TAAACCTAGA AGGAAAAGGG CAGCTATTCA    1860
   TGTTATGTTG GCTCTTGCAA CAGTATTATC TATTGCCGGT GCAGGGACGG GGGCTACTGC    1920
   TATAGGGATG GTAACACAAT ACCACCAAGT TCTGGCAACC CATCAAGAAT CTATGGAAAA    1980
   GGTGACTGAA GCCTTAGAGA TAAACAACTT AAGGTTAGTT ACATTAGAGC ATCAAGTACT    2040
   AGTAATAGGA TTAAAAGTAG AAGCTATGGA AAAATTTTTA TATACAGCTT TCGCTATGCA    2100
   AGAATTAGGA TGTAATCCAA ATCAATTTTT CTCCAAAATC CCTCTTGAGT TGTGGACAAG    2160
   GTATAATATG ACTATAAATC AAACAATATG GAATCATGGA AATATAACTT TGGGGGAATG    2220
   GTATAACCAC ACCAAAGATT TACAACCAAA GTTTTATGAA ATAATAATGG ACATAGAACC    2280
   AAATAATGTA CAAGGGAAAA CAGGGATACA ACAATTACCC AAGTGGGAAG ATTGGGTAAG    2340
   ATGGATAGGA AATATTCCAC AATATTTAAA GGGACTATTG GGAGGTATCT TGGGAATAGG    2400
   ATTAGGAGTG TTATTATTGA TTTTATGTTT ACCTACATTG GTTGATTGTA TAAGAAATTG    2460
   TATCCACAAG ATACTAGGAT ACACAGTAAT TGCAATGCCT GAAGTAGAAG GAGAAGAAAT    2520
   ACAACCACAA ATGGAATTGA GGAGAAATGG TAGCCAATTT GGCATGTCTG AAAAAGAGGA    2580
   GGAATGATGA AGTATCTCAG ACTTATTTTA TAAGGGAGAT ACTGTGCTAA GTTCTTCCCT    2640
   TTGAGGAAGG TATGTCATAT GAATCCATTT CGAACCAAAT CAAACTAATA AAGTATGTAT    2700
   TGTAAGGTAA AAGGAAAAGA CAAAGAAGAA GAAGAAAGAA GAAAGCTTTC AAGAGGATGA    2760
   TGACAGAGTT AGAAGATCGC TTCAGGAAGC TATTTGGCAC GACTTCTACA ACGGGAGACA 2820
   GCACAGTAGA TTCTGAAGAT GAACCTCCTA AAAAAGAAAA AAGGGTGGAC TGGGATGAGT 2880
   ATTGGAACCC TGAAGAAATA GAAAGAATGC TTATGGACTA GGGACTGTTT ACGAACAAAT 2940
   GATAAAAGGA AATAGCTAAG CATGACTCAT AGTTAAAGCG CTAGCAGCTG CTTAACCGCA 3000
   AAACCACATC CTATGTAAAG CTTGCTAATG ACGTATAAGT TGTTCCATTG TAAGAGTATA 3060
   TAACCAGTGC TTTGTGAAAC TTCGAGGAGT CTCTCCGTTG AGGACTTTCG AGTTCTCCCT 3120
   TGAGGCTCCC ACAGATACAA TAAATATTTG AGATTGAACC CTGTCAAGTA TCTGTGTAAT 3180
   CTTTTTTACC TGTGAGGTCT CGGAATCCGG GCCGAGAACT TCGCA 3225
(2) INFORMATION FOR SEQ ID NO:15:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (C) INDIVIDUAL ISOLATE: NCSU-1
   (viii) POSITION IN GENOME:
      (B) MAP POSITION: 857-880
      (C) UNITS: bp
   (xi) SEQUENCE DESCRIPTION: SEQ ID NO:15:
      TATGGAAAAG ATGGGATGAG ACTA 24
(2) INFORMATION FOR SEQ ID NO:16:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: feline immunodeficiency virus
      (C) INDIVIDUAL ISOLATE: NCSU-1
   (viii) POSITION IN GENOME:
      (B) MAP POSITION: 1513-1535
      (C) UNITS: bp
   (xi) SEQUENCE DESCRIPTION: SEQ ID NO:16:
      GTCACTTACC TTCATAGTAA ACC 23